# EUROPEAN PATENT SPECIFICATION

(11) **EP 1 996 088 B1**
(45) Date of publication and mention of the grant of the patent: **18.05.2011**
(21) Application number: 07706010.1
(22) Date of filing: 23.02.2007
(51) Int. Cl.: A61B 17/22, A61M 25/10, A61B 17/00, A61M 37/00, A61F 2/06

(54) **MINIMALLY INVASIVE INTRAVASCULAR TREATMENT DEVICE**
MINIMAL INVASIVE INTRAVASKULÄRE BEHANDLUNGSVORRICHTUNG
DISPOSITIF DE TRAITEMENT INTRAVASCULAIRE À INVASION MINIMALE

(30) Priority: 24.02.2006 EP 06003797
(43) Date of publication of application: 03.12.2008
(73) Proprietor: National University of Ireland Galway, Galway (IE)
(72) Inventor: MURPHY, Bruce, Philip, Galway (IE); LAWLOR, Vincent, Patrick, County Offaly (IE)
(74) Representative: Catesby, Olivia Joanne
(86) International application number: PCT/IE2007/000028
(87) International publication number: WO 2007/096856

(56) References cited:
- EP-B1- 0 850 031
- WO-A-98/22044
- US-A- 5 336 178
- US-A- 5 616 149
- US-A1- 2004 044 308
- US-A1- 2005 119 678

## Description

### Field of the Invention

The present invention relates to medical devices. In particular, the invention relates to a catheter based medical device for the treatment of internal body cavities such as arteries/veins or other hollow organs.

### Background to the Invention

Diseases of the circulatory system are the leading cause of death in the world, and the prevalence of the disease in younger patients is increasing. In addition, global society is following a trend whereby populations are exposing themselves to a greater extent to more of the risk factors associated with vascular disease.

For example, the rate of increase in obesity in Irish society was brought to public attention in a recent front-page Irish national newspaper article, "Tipping the scales: Child obesity levels triple" (The Irish Examiner 22/11/04). Furthermore, the statistics for cause of death present the true magnitude of the problem, in Ireland during the period 1998-2003 (inclusive) 40% of deaths within the state were caused by diseases of the circulatory system (source: Irish Central Statistics Office). This trend is not only a national problem, it is echoed internationally in 1998 in the United States 39% of all deaths were caused by diseases of the circulatory system (National Vital Statistics Reports 2000, Vol. 48, No. 11, July 24).

Atherosclerosis (vascular disease) is the accumulation of plaque within an artery wall. When the disease is at an advanced stage blood flow to organs, such as the heart, is reduced and as a consequence a heart attack or other acute event may occur. Balloon angioplasty was developed to reopen atherosclerotic arteries. This procedure involves inflating a miniature balloon at the site of an arterial blockage. Expansion of the balloon compresses the plaque and stretches the artery wall, this reopens the artery to its original diameter and restores blood flow (balloon angioplasty can be used on its own or as an adjunctive therapy to stenting). Angioplasty balloons are inflated to high pressures, up to 24atm (equivalent to 350 p.s.i. (2.4 x 10⁶ Pa) which is over 10 times the inflation pressure in an average car tyre). At these high pressures severe damage to the artery wall is caused. In a number of cases high pressure balloon angioplasty cannot dilate the blockage in the artery, specialist devices are then required to dilate the lesion, or bypass surgery is carried out.

This lead to the development of cutting balloons such as that disclosed in US Patent No. 5,196,024. This patent discloses a device and method for dilation or recanalisation of a diseased vessel by use of a balloon catheter with cutting edges to make longitudinal cuts in the vessel wall.

Since this first patent was filed, there has been considerable activity in the development of improved cutting balloons, with the emphasis on improving the blade-shielding capabilities of the cutting balloon.

In the balloon catheter disclosed in the aforementioned US Patent No. 5,196,024, the folds of the balloon in its collapsed state are used to shield the blades from the vessel wall during insertion and removal of the balloon catheter. One disadvantage of this arrangement is that the blades are not protected from damaging the balloon itself.

US Patent application number 2005/0137617 discloses a cutting balloon which aims to overcome this disadvantage. An elastically distensible folding member is disclosed which can be formed with a wall that is substantially shaped as a tube when the folding member is in a relaxed (i.e. unstressed) state. The tubular shaped folding member defines a tube axis and can have an axially aligned slit that extends through the wall. The folding member can be used to cover an incising element that is attached to the balloon and positioned in the lumen of the tubular folding member. During balloon inflation, the folding member can be deformed to expose the tip of the incising element to allow for a tissue incision.

US Patent Application No. 2005/0119678 or O'Brien et al. discloses an alternative solution wherein compressible sheaths made of a relatively low durometer, flexible material are mounted on the balloon to protect the operative cutting surface of a respective incising element during assembly of the cutting balloon and transit of the cutting balloon to the treatment site. Each sheath extends farther from the longitudinal axis than the corresponding incising element and makes first contact with the tissue during a balloon inflation. Once contact has been established between the tissue and the sheath, further balloon inflation causes the sheath to radially compress between the tissue and the inflatable balloon exposing the operative cutting surface for tissue incision.

US Patent Application No. 2004/0133223 of Weber also discloses the use of a resilient material which extends over the cutting edge of a blade on a cutting balloon, the resilient material deforming under compression to allow the cutting edge to pierce through.

The aforementioned US Patent No. 5,196,024, also discloses the use of a protective sheath which covers the entire balloon. Continuity of the sheath is interrupted by longitudinal grooves which serve to accommodate, guide and protect the tips of the (balloon's) cutting edges. The protective sheath prevents vessel injuries during delivery and holds the cutting edges in proper position prior to balloon inflation. As the balloon is inflated, the grooves of the protective sheath open up allowing the cutting edges to penetrate into the vessel wall producing cuts with sharp margins. After deflation, the cutting edges retract behind the protective sheath thereby avoiding injury to the vessel during withdrawal of the cutting balloon. An alternative solution to the problem of exposed blades damaging the balloon is disclosed in one embodiment in US Patent No. 5,196,024 wherein the blades are repositioned onto a plastic casing surrounding the balloon. Continuity of the casing is interrupted by longitudinal slots which increase in size as the balloon is inflated.

A similar arrangement is disclosed in US Patent No. 5,797,935, wherein a balloon activated force concentrator for use in cooperation with an inflatable angioplasty balloon includes at least one elongated flexible panel, an elongated cutting blade mounted on the outside surface of the elongated flexible panel, and an elastic circular band attached to each end of the elongated flexible panel for securing the elongated flexible panel to an angioplasty balloon.

Cutting balloons such as those discussed above are now commonly used on highly calcified lesions or stubborn lesions, sometimes on their own or prior to stent placement. However, these devices have been found to be prone to failure, are relatively large and difficult to manoeuvre within the vasculature, and are often restrictively expensive.

One of the greatest problems is associated with the removal of the cutting balloon after inflation. The pressure of the balloon can in some cases cause the cutting edges or blades to penetrate deeply into the vessel wall. To subsequently withdraw the blades can require a strong force. In each of the above examples of cutting balloons, it is the balloon itself upon deflation which-provides this retraction force. It has been known for difficulties in retracting the blades to occur, and in extreme cases removal of the cutting balloon has been impossible, resulting in a cutting balloon being left in a patient's coronary artery possibly due to being caught in a (previously implanted) stent.

What is required therefore is an alternative to existing cutting balloons that will be more efficient, easier to use and safer.

As discussed above, cutting balloons are used to reopen blocked vessels, typically resulting from vascular disease. However, cutting balloons do not address the treatment of such vascular disease. With a continuing trend of people dying from vascular disease, and young patients increasingly exposing themselves to obesity together with the associated increased risk of diabetes, innovative effective therapies must be conceptualised to treat both the younger and the traditional older sufferer of vascular disease. These trends, along with technological advances, have resulted in an annual growth rate of approximately 20% in transcatheter technologies.

One of the main drivers of this growth rate is coronary drug eluting stents; however there are a number of areas where these stents cannot be used effectively; namely, chronic total occlusions, peripheral artery disease, and vulnerable plaque. Furthermore new devices and treatments are needed to treat restenosis associated with the edge of drug eluting stents and in-stent restenosis associated with bare metal stents. All of the above mentioned areas represent significant unmet clinical needs as no technology can adequately treat these conditions.

Advances in local drug delivery have proven extremely effective in the coronary arena, whereby drug-eluting stents have made a significant breakthrough in the prevention of in-stent restenosis. In the Boston Scientific sponsored TAXUS IV trial, which compared the TAXUS SR drug eluting stent on the Express-1 platform to an identical bare metal Express-1 stent, it was demonstrated that in-stent restenosis at 9 months can be reduced from 24.4% for the bare metal stent to 5.5% by using an equivalent drug eluting stent (Journal of Interventional Cardiology 2004; Vol. 17, No. 5, p279). Local drug delivery rather than systemic therapy has provided excellent results in the case of coronary drug-eluting stents; future therapies such as gene therapy and stem cell therapy require some form of local delivery device, as these therapies involve the time consuming production of expensive, minuscule quantities of molecules/compounds. A systemic non-efficient approach would not be cost effective for gene therapy, as most of the molecules/compounds would not reach the required target site - a different more efficient approach is required.

The state of the art at present for atherosclerosis, and in particular treating blocked coronary arteries, involves the implantation of a drug eluting coronary stent. This action re-establishes blood flow to ischemic areas of the heart muscle. However, there are certain situations caused by different stages of the disease or vascular disease affecting different blood vessels where a stent cannot be implanted. In these situations a different strategy must be adopted. Future therapies, such as biotherapeutic local delivery for molecular cardiology and molecular vascular intervention, are on the forefront of clinical medicine and promise to provide therapeutic treatment for the next generation of patients. These new treatment methods could make a difference to the quality of life of patients who have the following conditions:

### • Chronic Total Occlusions (CTO).

A CTO is a complete obstruction of an arterial lumen and it is estimated that 10-20% of all coronary angioplasty procedures involve a CTO (Freed and Safian, The Manual of Interventional Cardiology, 3rd ed; p287). CTOs can occur in other arteries, for example femoral arteries. A CTO in a femoral artery restricts blood flow to the remainder of the patient's leg and may cause critical limb ischemia, and consequently ulcerations and gangrene can occur and in some cases amputation is necessary. In addition slight angiogenesis (formation of new blood vessels) may occur allowing small amounts of blood to reach the lower leg. Angiogenesis in some cases may be crucial for survival. The process of angiogenesis can be artificially accelerated by injection of Vascular Endothelial Growth Factor (VEGF), this was demonstrated in an animal model of CTOs. Nikol et al. (Acta Physiologica Scandinavica 2002, Vol. 176, Iss. 2, p151) showed that injection of VEGF significantly increased the number of artery branches and the area of branches in a pig model of CTOs. With encouraging results from animal models it is expected that this form of gene therapy for CTOs will be transferred to a clinical application in the near future, if this occurs physicians would require a safe efficient catheter for delivery of the therapeutic solution.

### • Peripheral Artery Disease (PAD)

PAD is a condition similar to coronary artery disease. In PAD, fatty deposits build up in the inner linings of the artery walls, mainly in arteries leading to the kidneys, stomach, arms, legs and feet. This causes dysfunction of individual organs or limbs. PAD is slightly different to coronary artery disease as it affects arteries near to the surface of the body compared to the well-protected (from external mechanical loads) arteries of the heart. Stainless steel or cobalt chrome stents cannot be used safely in PAD because if they experience an excessive external load they will not retain their shape due to plasticity of the material. An external load in this case would cause an instantaneous obstruction within the artery lumen and consequent loss of blood flow. The challenging anatomy of peripheral arteries, the prevalence of long total occlusions, and a number of unique mechanical loads all lead to high restenosis rates in femoropopliteal and infrapopliteal interventions and patients with superficial femoral artery stenoses have patency rates of less than 50% at 1 to 3 years clinical follow-up (Radiology, 1994; 191; p727-733). Stents appear to be an inadequate treatment option for peripheral arteries and additional methods and treatment strategies for peripheral interventions that do not rely on a mechanical solution for the biological problem must be employed, i.e. local delivery of therapeutic products to these lesions.

### • Stent edge restenosis and in-stent restenosis

There is a potential for local biotherapeutic delivery to the edge of Bare Metal Stents (BMS) and Drug Eluting Stents (DES). In a study by Serruys *et al.* significant restenosis rates at the proximal edge of DES and BMS were reported in an IVUS study, at 6 months follow-up after stenting a significant decrease in proximal lumen area was observed for slow release, medium release TAXUS eluting stents and bare metal stents (Circulation 2004, Vol. 109, p627-633).

### • Vulnerable plaque

Vulnerable plaque is a type of lesion that is buried inside the artery wall and may not always bulge out and block blood flow; it is now an accepted fact that this type of plaque accounts for the vast majority of acute coronary syndromes (Cardiovascular Research 1999, Vol. 41, p323-333). Vulnerable plaque is asymptomatic and difficult to diagnose with present technology. However, advances in screening techniques and diagnostic technology (Virtual Histology IVUS and thermography catheters) allow these lesions to be identified. This type of lesion is non-stenotic and does not require a mechanical solution, it would be more advantages to change the function of the tissue by delivering a biotherapeutic solution to the lesion site.

Numerous catheter based local therapeutic delivery devices for the delivery of gene therapy products (or drugs) directly to target sites within a vessel or artery have been developed.

United States Patent No. 6,048,332 (Duffy, et al.) entitled "Dimpled porous infusion balloon" discloses drug delivery catheters that have dimpled porous balloons mounted onto the distal end of the catheter. In one embodiment, the balloons are adapted for delivering therapeutic agents to the tissue wall of a body lumen, and to this end include a plurality of dimples formed in the exterior surface of the balloon, with each dimple having at least one aperture through which a fluid delivered into the interior of the balloon can extravasate. It is understood that the balloons described therein provide, inter alia, increased coverage of the tissue wall to which the agent is being delivered and less traumatic contact between the agent being delivered and the tissue wall.

United States Patent No. 5,336,178 (Kaplan, et al.) discloses an intravascular catheter with an infusion array. An intravascular catheter provides means for infusing an agent into a treatment site in a body lumen and means for deploying the infusing means adjacent the treatment site, which operate independently of one another. In one embodiment, a flexible catheter body has an expansion member attached to its distal end in communication with an inflation passage, and an infusion array disposed about the expansion member in communication with one or more delivery passages. The infusion array includes a plurality of delivery conduits having laterally oriented orifices. The delivery conduits may be extended radially from the catheter body to contact a treatment site by expanding the expansion member with an inflation fluid. An agent may be introduced into the delivery passages and infused into the treatment site through orifices in the delivery conduits. The expansion member may be expanded for dilatation of the lumen before, during, or after infusion.

United States Patent No 6,369,039 (Palasis et al.) entitled "High efficiency local drug delivery" discloses a method of site-specifically delivering a therapeutic agent to a target location within a body cavity, vasculature or tissue. The method comprises the steps of providing a medical device having a substantially saturated solution of therapeutic agent associated therewith; introducing the medical device into the body cavity, vasculature or tissue; releasing a volume of the solution of therapeutic agent from the medical device at the target location at a pressure of from about 0 to about 5 atmospheres for a time of up to about 5 minutes; and withdrawing the medical device from the body cavity, vasculature or tissue. One problem with this device is its low delivery pressures.

The above are all examples of infusion catheters, with no needles involved. In vivo studies show that these catheters have inferior clinical results in comparison to other drug delivery methods. Infusion has been shown to be an inferior drug delivery method to needles.

United States Patent No. 5,112,305 (Barath, et al.) entitled "Catheter device for intramural delivery of therapeutic agents" discloses a method of treatment of an atherosclerotic blood vessel. Specifically, therapeutic agents are delivered by means of a specialized catheter system to the deeper layers of the vessel wall with only minimal interruption of the vessel endothelium. This system will allow high local concentrations of otherwise toxic agents directly at the site of an atherosclerotic plaque. The catheter system and method will deliver chemical agents intramurally at the precise vessel segment that is diseased but without allowing the agents to diffuse distally into the bloodstream. One embodiment disclosed employs a double lumen catheter that has additional tubular extensions projecting at various angles from the outer surface of the outermost lumen. By abruptly increasing the pressure in the outer lumen, the tubular extensions deliver the therapeutic agent to locations deep within the vessel wall.

This an example of an early device employing needles at a time when technology to join balloons and needles was undeveloped. Furthermore, the balloon needs to be inflated when it is not airtight due to the holes associated with the protrusions, which is not sensible and could cause problems with excessive therapeutic agents transferred to the blood stream rather than the target site. There may also be problems with balloon deflation.

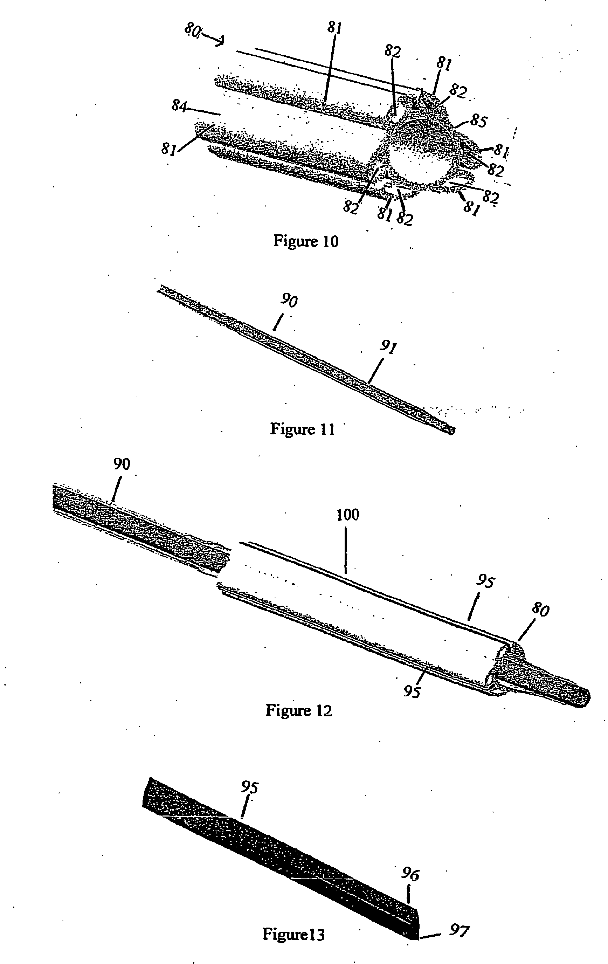

Barath also describes in later US Patent No. 5,615, 149 a balloon catheter with a cutting edge. A sheath is provided in one embodiment (see Figures 12 and 13). In common with Naimark *et al* (see below) the balloon must be expanded before the sheath is contacted.

United States Patent No. 5,873,852 (Vigil, et al.) entitled "Device for injecting fluid into a wall of a blood vessel", discloses a method and device for injecting fluid into a treatment area of a vessel wall. A first version of the device includes an inflatable balloon mounted on a catheter and a plurality of injectors extending outwardly and moving with the balloon. At least one fluid passageway connects each injector in fluid communication with a fluid source. During use of the device, the balloon is first positioned in a vessel proximate the treatment area. Next, the balloon is inflated to embed the injectors into the vessel wall. Subsequently, the fluid from the fluid source is introduced into the fluid passageway and through the injectors into the treatment area.

It will be appreciated therefore that the needles are free to cause damage to the endothelial surface upon delivery and retraction of the device.

United States Patent No. 5,354,279 (Hofling) entitled "Plural needle injection catheter" discloses a catheter for the injection of a fluid, for example, medicine, into body cavities such as veins or other hollow organs. The catheter is provided with a head which is insertable into the body cavity and includes hollow needles movably disposed therein between retracted and extended positions and with an operating mechanism mounted to the end of the catheter opposite the head and operatively connected to the needles for moving their front ends outwardly in contact with the walls of the body cavity for supplying the fluid or medicine through the hollow needles directly to the wall portions of the body cavities to be treated. A balloon may be disposed in front of the catheter head and may be inflated or deflated by way of a passage extending through the catheter. This needle injection catheter is awkward to use and requires additional steps that need precision control by the operator and may be prone to some form of error. Unpredictable advancement of the needle due to the difficult to control needle advancement mechanism might occur, and vessel perforations are possible, both of which are highly undesirable.

United States Patent No. 6,197,013 Reed, et al.) entitled "Method and apparatus for drug and gene delivery" discloses an apparatus and method for treating a patient. The apparatus includes a deployment mechanism having a surface. The apparatus also includes at least one probe disposed on the deployment mechanism surface. The probe extends between 25 microns and 1000 microns from the surface of the deployment mechanism. The apparatus also includes material coated on the probe. The method of treatment includes the steps of placing a material with a probe which extends less than 1000 microns from a surface of a deployment mechanism. Next, there is the step of inserting the probe into preferably a blood vessel of a patient. Then, there is the step of penetrating the interior wall of the vessel from the interior of the vessel with the probe by activating the deployment mechanism so the material can contact the vessel.

A problem with this arrangement is that the sharp probes on the outside of the stent or the catheter may cause damage during delivery or removal of the stent, although there is a mention of a protective sheath that is removed prior to dilation.

United States Patent No. 6,283,947 (Mirzaee) entitled "Local drug delivery injection catheter" discloses a catheter for injecting medication to a specific point within a patient comprises a drug delivery lumen extending from a proximal end of the catheter to an injection port. The catheter comprises a mechanism for angularly pushing the injection port outwardly away from the body of the catheter into an artery wall so that medication can be injected directly into the artery wall. The catheter comprises an injection port at or near the distal end thereof and a mechanism for directing the injection port angularly away from the central axis of the catheter and into the artery wall. (An injection port is a structure used for introducing medication or other material into a patient. The injection port typically is a hollow needle.) In one embodiment, the catheter includes a guide wire lumen for receiving a guide wire that enables a physician to direct the catheter to a desired location within the patient's vascular system. Also, in one embodiment, the catheter includes a plurality of needles, each of which may be manipulated at an angle outwardly from the central longitudinal axis of the catheter so that the needles can inject a drug or medication into the surrounding tissue. Prior to deployment of the needles, the needles are retained such that they lie substantially parallel to the longitudinal axis of the catheter. In one embodiment, a balloon is provided towards the distal end of the catheter for pushing the needles outwardly into the artery wall. In another embodiment, other mechanical means are provided for pushing the needles outwardly.

Problems experienced by this device include operational difficulties, difficulties with advancing sheath after use, and lack of flexibility.

United States Patent No. 6,494,862 (Ray, et al.) entitled "Substance delivery apparatus and a method of delivering a therapeutic substance to an anatomical passageway" discloses a catheter assembly having a balloon disposed at the distal end thereof. The balloon is capable of being inflated to selectively dilate from a collapsed configuration to an expanded configuration. A syringe assembly is in fluid communication with a delivery lumen of the catheter assembly for allowing a therapeutic substance to be injected into a tissue of a passageway. The syringe assembly includes a portion capable of pivoting from a first position towards a second position when the balloon is being inflated from the collapsed configuration to the expanded configuration. The portion of the syringe assembly is also capable of pivoting from the second position back towards the first position when the balloon is being deflated. One problem with this device is that the pivoting may cause ripping/damage of the inner artery wall.

United States Patent 6,695,830 (Vigil, et al.) entitled "Method for delivering medication into an arterial wall for prevention ofrestenosis" discloses a method for preventing a restenosis within a vessel wall, wherein a medicament is required to be delivered at predetermined locations into the vessel wall and allowed to subsequently disperse in a predetermined pattern. To deliver the medicament, a catheter with an expanding member is advanced into the vasculature of a patient until the expanding member is located as desired. The expanding member is then expanded to force dispensers into the vessel wall to the proper depth. A medicament is then pumped through the dispensers to create a plurality of equally spaced, localized medicinal deliveries which subsequently disperse to medicate an annulus shaped volume within the vessel wall.

Naimark *et al* in US Patent Publication No. US 2004/0044308 describe an apparatus for the delivery of biologically active materials which includes a catheter, a balloon, microneedles on the balloon and which can further include a sheath. The sheath is described as being made of metals. One alternative discussed is to make the sheath of expandable material. The sheath optionally has a plurality of ports for the microneedles or is made of a material capable of being punctured by those needles. The balloon of the Naimark *et al* device is inflated it moves out to contact the sheath and the sheath may, once contact is established, expand with the balloon. This construction can be seen for example from Figure 5a of that document. Having the sheath spaced radially outward and apart from the microneedles (in Barath (above) outward of the blades) ensures protection for the vessel wall from scraping when the balloon is unexpanded.

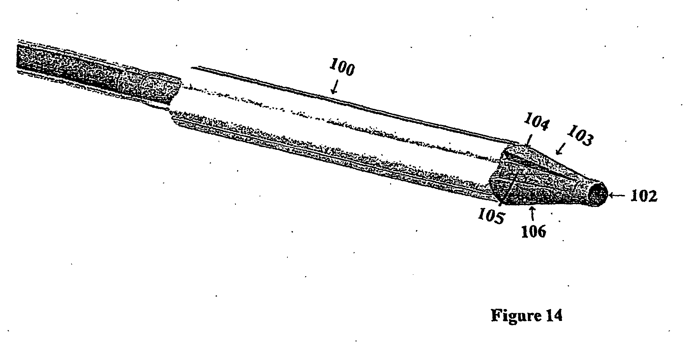

US 5,336,178 (Kaplan et al) describes an intravascular catheter for infusing an agent into a treatment site. It employs a series of apertures to infuse the liquid agent. An internal elastomeric sleeve is described in certain embodiments (see Figures 13 and 14A). The device does not have to deal with treatment implements such as needles or cutting blades.

US Patent No. 6,051,001 (Borghi), EP 0 697 226 (Igaki), US 6,018,857 (Duffy et al) and WO 98/22044 all describe devices for loading of stents for example onto a catheter.

It will be appreciated that current devices for delivering therapeutic agents to the arterial wall or for providing a cutting action experience problems either with safety or efficiency. This is due in part to the difficulties in introducing (sharp) working implements into a body, for example a body lumen, in a state where the implements do not contact a vessel wall during insertion or removal but which can be deployed to contact a target area of the vessel wall and thereafter returned, after use, to a position where the device can be removed from the vessel without the implements contacting the vessel wall to allow safe removal from the body. Furthermore, the current devices are limited in their areas of application. A further problem commonly experienced by current devices is incomplete balloon deflation or deflation failure. This causes a serious safety issue as it is essential that the balloon can deflate quickly and completely to allow removal of the catheter from the vessel without causing subsequent damage to the vessel wall.

Accordingly, what is required is a sheath for a local catheter based therapeutic delivery device that allows treatment implements such as needles or blades to be concealed when the catheter is being manoeuvred into position, to permit safe delivery of the device to the desired treatment area, without causing damage to the inner lining of the artery wall during delivery.

### Object of the Invention

It is an object of the invention to provide an efficient and effective sheath for a catheter based local therapeutic device which may be adapted for the delivery of gene therapy products (or drugs) directly to target sites, and/or which may be provided with cutting implements which can be used to treat a site within the body.

It is a further object of the invention to provide a sheath for a local catheter based therapeutic delivery device capable of use in a number of product applications.

It is a further object of the invention to provide a sheath for a delivery device which can be used at more than one site of treatment within a vessel/artery. This feature is particularly useful in diffuse peripheral disease or for arteries with numerous vulnerable plaques.

It is a further object of the invention to provide a sheath for a delivery device which experiences quick and safe deflation after use.

It is a further object of the invention to provide a sheath for a delivery device with sufficient flexibility so as two allow the catheter to navigate tortuous arteries.

It is a further object of the invention to provide a sheath for a delivery device wherein drugs may be delivered (and thus distributed) evenly compared to catheters available at present.

### Summary of Invention

The present invention relates to a sheath for fitting to a balloon catheter as set out in the appended claims. The sheath of the present invention is described herein with reference to a device for treating a targe area of a vessel wall of a vessel within a human or animal body, the device comprising:
(a) an expandable portion for radially expanding the device from a contracted configuration allowing travel within the vessel to the target area to an expanded configuration allowing treatment of the target area;
(b) a protective sheath stretch-fitted over the expandable portion to exert a compressive force on the expandable portion for radially contracting the device from its expanded configuration to its contracted configuration, and for exerting a compressive force on the expandable portion in its contracted configuration, and
(c) at least two spaced apart treatment implements extending radially outwardly from the expandable portion, wherein in the device's contracted configuration the implements are shielded within the protective sheath, and in its expanded configuration the thickness of the sheath decreases to expose the implements for contact with the target area of the vessel wall.

The device thus provides a simple yet efficient construction which obviates many of the problems associated with the prior art described above including non-collapse of the expandable portion following use.

The pre-stretched configuration of the sheath on the non-expanded configuration of the expandable portion is sufficient to return the expandable portion to a non-expanded configuration. Generally the sheath will be constructed so that it must be (pre-)stretched by at least 10%, more desirably at least 12% such as at least 15% so as to overfit the non-expanded configuration of the expandable portion. There is thus potential energy in the (elastic) stretch-fit of the expandable member.

Preferably the expandable portion is a balloon. This is a simple yet effective construction.

In one embodiment of sheath the treatment implements may be blades for cutting or scoring the vessel wall. Alternatively, the treatment implements may take as different form, for example needles (such as hollow needles or micro-needles) wherein the device may act as a drug delivery device for the delivery of therapeutic substances to the vessel wall. When needles are used, preferably the device further comprises a drug delivery system in fluid communication with the needles for delivery of therapeutic compound through the needles into the vessel wall. The drug delivery system may comprise a plurality of reservoirs in the protective sheath. Alternatively, the drug delivery system may comprise a (multi-lumen) supply hose connected via (flexible) tubing to the needles. The sheath thus provides the opportunity to adapt a balloon catheter into a device with one or more implements for treating target sites.

Preferably the protective sheath comprises an elastic polymer, such as silicone or a polyurethane material or rubber. Polyurethane may allow more options in fixing an implement to a sheath. Preferably the protective sheath has defined therein a plurality of holes in which or beneath which the treatment implements are seated.

The device may further comprise at least one marker (such as a radiopaque marker) to aid positioning of the device. This allows the position of the device to be monitored closely.

The device may be fitted with a nose-cone. The nose-cone provides a transitional profile between the catheter and the sheath on a leading end thereof. This means that during forward travel the device is less likely to encounter resistance to travel due to the difference in size (diameter) of the catheter and a sheath mounted thereon. The nose - cone will allow for more gradual stretching of the vessel in which the device is traveling. Similarly for retraction of the device from its working position a tail-cone may be provided which provides a transitional profile between the catheter and the sheath on the trailing end thereof. This again allows for ease of retraction.

The protective sheath may be fitted to a device for treating a target area of a vessel wall of a vessel within a human or animal body, the device comprising:
a) an expandable portion for radially expanding the device from a contracted configuration allowing travel within the vessel to the target area to an expanded configuration allowing treatment of the target area,
b) at least two spaced apart treatment implements extending radially outwardly from the expandable portion,
the protective sheath adapted to be fitted (optionally stretch-fitted) over the expandable portion to exert a compressive force on the expandable portion for radially contracting the device from its expanded configuration to its contracted configuration,
wherein in the device's contracted configuration the implements are shielded within the protective sheath, and in its expanded configuration the thickness of the sheath decreases to expose the implements for contact with the target area of the vessel wall. Generally the expandable portion will be already under contraction force from the sheath or will immediately, upon expansion experience contraction force from the sheath.

According to an example the invention there is further provided a sheath for fitting to a balloon catheter for treating a target area of a vessel wall of a vessel within a human or animal body, the sheath adapted to be stretch-fitted over the balloon to exert a compressive force on the balloon for radially contracting the balloon from its expanded configuration to its contracted configuration, the sheath comprising:
at least two spaced apart treatment implements mounted within the sheath so as to extend radially outwardly from the balloon, wherein in the balloon's contracted configuration the implements are shielded within the sheath, and in its expanded configuration the thickness of the sheath decreases to expose the implements for contact with the target area of the vessel wall.

It will be appreciated that to overfit an expandable member such as a balloon the sheath will have an annular (wall or body) construction. It is desirable that the sheath is substantially continuous in an annular direction. If for example the sheath were discontinuous in an annular direction, for example slotted to any substantial extent, the effect during expansion may be for the discontinuity (slot) to become greater, for example slot(s) widen. In such a case the thickness of the sheath may not decrease to expose the implements for contact with the target area of the vessel wall.

It will be appreciated in such embodiments that the sheath acts as a carrier for the treatment implements, which may be coupled or mounted on or within the sheath. Preferably the sheath comprises an elastic polymer, such as silicone. Generally the implements will be mounted so as project outwardly from the sheath. The implements will not generally be mounted directly to the expandable member. This arrangement obviates the problem of implement/expandable member interaction which can in turn be responsible for device failure due to puncturing, snarling etc.

In one embodiment, the treatment implements may be one or more needles for example hollow needles. The sheath may then further comprise:
an inner sheath comprising an outer surface on which a plurality of reservoirs are provided for storing therapeutic compound; and
an outer sheath positioned over the inner sheath;
wherein the needles each comprise a base portion and an injector portion, and wherein each base portion is located over a reservoir on the outer surface of the inner sheath, and wherein each injector portion extends radially outwards from the inner sheath and is received through cooperating holes defined within the outer sheath.

When treatment implements are needles, the sheath may be used to convert a standard balloon catheter into a catheter based drug deliver device.

In an alternative embodiment the treatment implements may be cutting implements for example blades, or microsurgical scalpels. The sheath preferably contains a number of microsurgical scalpels on its outer surface. These scalpels may be initially concealed from the artery wall by the external contours of the sheath.

The sheath may comprise at least one protuberance on its outer surface, wherein in each protuberance extends further radially outwardly from the outer surface of the sheath than each cutting implement.

Preferably each protuberance is collapsible. In a preferred embodiment each protuberance has a hollow internal pocket (a hollow centre), wherein in the balloon's expanded configuration the deformation of the sheath causes the pocket to flatten out thereby reducing the size of the protuberance in the radial direction to expose each cutting implement. The protuberance therefore becomes flattened as the sheath deforms with inflation of the balloon. When the balloon is inflated the contours of the sheath become smooth and the cutting edges are exposed. Moreover the sheath allows optimum balloon folding and minimum balloon withdrawal resistance leading to a safer and easier to use device. The (silicone) sheath has a number of functions, (i) it protects the artery wall from the implements (scalpel blades) when the catheter is being manoeuvred in to position, (ii) it prevents balloon/implement (blade) direct contact so the balloon cannot be dissected by a blade, (iii) keeps all the implements (blades) perpendicular to the balloon at all times, (iv) aids deflation of the balloon to its original profile which subsequently reduces balloon withdrawal resistance, (v) the sheath allows optimum folding of the balloon which will reduce the profile of the catheter when compared to present technology.

It will be appreciated that when the treatment implements are blades, the sheath may be used to convert a standard angioplasty balloon into a cutting balloon.

The sheath may further be provided with at least one marker such as a radiopaque marker to aid positioning of the sheath.

The protuberances may be provided in pairs and desirably at least one pair of protuberances are provided - each on opposing sides of the treatment implement. This ensures effective shielding of the implements. Desirably the at least one protuberance has a curved exterior surface. This curved profile again allows for ease of movement of the device with the vessel - there are no angular shapes for catching/snagging. In this respect having the curved exterior surface as a convex surface is useful.

The present inventors have found that one suitable construction which provides effective shielding but which also is of a shape suitable for travel within a vessel etc. is where the at least one protuberance is substantially elliptical in its cross-sectional shape. It has been found that such shapes provide effective shielding yet collapse effectively to an essentially circular configuration. Desirably the pair of protuberances converge toward each other and to a point above the working implement. This profiling toward the implement allows effective shielding yet effective retraction of the protuberances (resulting in an overall substantial decrease in thickness of the sheath).

Where pairs of protuberances are provided the pairs of protuberances may be substantially elliptical in its cross-sectional shape.

As stated above it is desirable that in an expanded configuration, the sheath including its at least one protuberance assumes a substantially circular shape when the protuberance flattens. Essentially this means that the thickness of the sheath reduces from that of the unexpanded sheath/protuberance to that of the expanded sheath/flattened protuberance.

In accordance with the invention, a base end of the implement is recessed into the sheath. Desirably the implement is a cutting implement and a base end of the cutting implement is recessed into the sheath. This means for example the implement can be moulded into the sheath when the sheath is being formed. In order to avoid dislodgement of the implement from the recess (e.g. due to stretching of the recess) it is desirable that a stretch-resistant element is provided on the sheath proximate the recessed cutting implement, for example below the cutting implement, so as to prevent local stretching of the sheath.

It will be appreciated that the sheaths of the present invention generally take the form of an annular ring of material.

As one alternative or as an addition to having an external profile which is interrupted due to the presence of protuberances projecting from the annular ring of the sheath, the present inventors have found that is useful to form within the ring at least one hollow internal pocket, wherein, in the balloon's expanded configuration, the deformation of the sheath causes the pocket to flatten out. The presence of the pocket may mean that the thickness of the ringer may be greater, but nonetheless the outer profile is not interrupted by protuberances.

A treatment implement may be housed within at least one hollow pocket, and in the balloon's expanded configuration, the deformation of the sheath causes the pocket to flatten out so as to expose the treatment implement for use. This is an internal housing within the pocket, with the pocket extending across the implement so that the implement does not extend beyond the outer profile of the pocket. The implement is thus very effectively shielded. Optionally the pocket is provided with an aperture through which the working implement extends in the balloon's expanded configuration.

It will be appreciated that a plurality of pockets may be provided, each housing a working implement. However it may be desirable to alternatively or additionally provide (within the ring of material) at least one pocket is provided which does not house a working implement. Such a pocket could be used as a control pocket to control the reduction in thickness of the sheath. Such pockets would generally be placed proximate a working implement to ensure a greater reduction in thickness of the sheath. This in turn may allow for greater exposure of the implement. It may be desirable to provide a plurality of pockets are provided each of which does not house a working implement.

It will be further appreciated that any sheath of the present invention may be assembled for operation on a catheter having an expandable member such as a balloon catheter.

Accordingly, there is provided a sheath for fitting to a local catheter based treatment device for use as a therapeutic substance delivery device or a cutting device, based on a technology platform that utilises an efficient and safe technology to treat sites of disease/damage within a blood vessel wall. The technology is a catheter-based system that utilises the material properties of a soft sheath (made from, for example, silicone /or custom microstructural material) to conceal treatment implements (such as injection needles) from the artery wall when the catheter is being advanced to its site of use.

When the catheter is located at its intended site of use a balloon is inflated. In an embodiment wherein the treatment implements are needles, this forces a series of needles outwards in the radial direction; the balloon expansion causes the sheath to stretch over the balloon, and the needles, which are located between the balloon and sheath, are pushed through holes located in the sheath and onwards into the site of disease or desired area of drug delivery in the artery wall.

The device relies on this principle to conceal the needles initially and secondly to utilise the incompressible material properties of the sheath to allow the needles to be exposed at the site of therapeutic delivery when the balloon is inflated. The technology offers a safe methodology to deliver therapeutic agents as the catheter will cause minimal damage to the artery wall when it is being placed in position.

A diffuse needle arrangement allows the drugs to be distributed evenly compared to catheters available at present. Minimum damage is caused to the artery wall by this method thus neointimal hyperplasia should not be a significant problem with the device of the present invention.

It will be appreciated that the device can be used at more than one site as the sheath causes the balloon and the needles to retract into their original position. Following this, the device could be moved to the next site of treatment. This feature could be useful in diffuse peripheral disease or for arteries with numerous vulnerable plaques. This feature also reduces the balloon withdrawal resistance of the device.

It will further be appreciated that the sheath also protects the balloon against contact with the implements. Contact between the implements and the balloon is undesirable as could cause puncturing of the balloon.

The primary advantage of the device is the manner in which the treatment implements are concealed within the catheter and the manner in which the material properties of the sheath are used to reveal the implements at the correct location.

Moreover, using this device, the method of drug delivery is more efficient than methods available at present

It is these two aspects that differentiate the invention from products available, and patented products that are not in clinical use at present. Previous designs incorporated exposed needles, which could cause damage to the artery wall, and previous local drug delivery catheters were never very efficient, delivering only approximately 15% of the drug to the desired area.

The approach taken by the device will always cause balloon deflation after a procedure, as the elastic sheath will produce automatic balloon deflation and retraction of the needles. This removes any doubt of issues of balloon deflation. Prior art devices do not have this fail-safe mechanism.

Further differences between the invention and the prior art include:
1. The sleeve always fits tightly on the balloon in both the retracted and expanded positions.
2. The elastic material is used to conceal implements
3. The sheath can be retrofitted to any balloon catheter.

Furthermore, the invention may be used as a platform technology for a number of different applications, either as a stand alone device or as an additional feature of a current procedure e.g. a module to prevent proximal or distal restenosis during delivery of a drug eluting stent.

The technology could provide a significant commercial return as current devices for delivering therapeutic agents to the arterial wall, and devices for dilation of diseased vessels are not as safe or as efficient as the proposed platform technology, furthermore the current devices are limited in their areas of application while this present technology platform has been designed so that a number of product applications are possible.

It will be appreciated that the geometry and design of the device may be adapted to suit its intended application. For example, when used as a chronic total occlusion catheter, all the needles will be weighted towards the front of the catheter, the profile will be modified slightly and a specific balloon geometry will be used to account for the lesion geometry.

The device may also be used for local biotherapeutic delivery to the edge of Bare Metal Stents (BMS) and Drug Eluting Stents (DES).

A sheath according to the present invention may be incorporated a stent delivery catheter. The design of this module will not compromise the cross-ability or the profile of the stent delivery catheter. On BMSs, use of the invention in this manner may reduce in-stent restenosis. This module would allow direct injection into the artery wall of anti proliferative drugs without the need to develop complex and costly drug eluting polymer coatings.

For a drug delivery module located on a DES it is expected that the material properties or geometry will have to be altered slightly to match that of the stent expansion so that a single balloon could be used for the entire delivery (stent and drugs), the drugs could be injected as the stent is being held in place by the cardiologist.

The present invention could be used to deliver the biotherapeutic solution to the lesion site.

### Brief Description of the Drawings

The present invention will now be described in greater detail with reference to the accompanying drawings in which:
Figure 1 is a representation of a device.
Figure 2 is a sectional representation of a device during operation in-vivo within a vascular cavity.
Figure 3 a is a side cross sectional view of the device of Figure 2 pre balloon deployment.
Figure 3b is an end cross-sectional view of the device of Figure 3a taken along line A-A' in Figure 3a.
Figure 4a is a side cross sectional view of the device of Figure 2 post balloon deployment and drug delivery.
Figure 4b is an end cross-sectional view of the device of Figure 4a taken along line A-A' in Figure 4a.
Figure 5 is a set of perspective views of three further examples of devices.
Figure 6 is a perspective representation of a sheath according to one embodiment of the invention.
Figure 7 is a cross-sectional view of a device in accordance with the invention in its expanded configuration.
Figure 8 is a cross-sectional view of the device of Figure 7 in its retracted configuration.
Figure 9a is a cross-sectional view of a cutting sheath.
Figure 9b is a close-up view of a blade region of the sheath of Figure 9a.
Figure 9c is cross-sectional view of a portion of the sheath of Figure 9a in its deformed state with the blade exposed.
Figure 10 is a perspective view of an alternative sheath construction.
Figure 11 is a perspective view of a balloon catheter.
Figure 12 is a perspective view of an assembly comprising the sheath of Figure 10 mounted to the catheter of Figure 11.
Figure 13 is a perspective view of one embodiment of a cutting implement which may be used within the present invention.
Figure 14 shows a perspective view of an assembly according to Figure 12 further comprising a nose cone.
Figure 15 shows a cross-sectional view of the sheath of Figure 10 in an unexpanded configuration.
Figure 16 shows a cross-sectional view of the sheath of Figure 10 in a partially expanded configuration.
Figure 17 shows a cross-sectional view of the sheath of Figure 10 in a fully expanded configuration.
Figure 18 shows the reversible sequence (indicated by the double-headed arrow) of

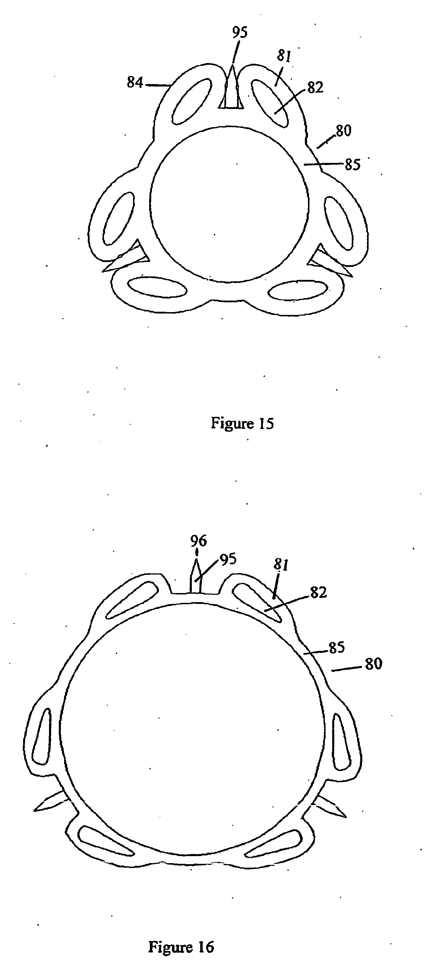

Figures 15 through 17 in a single Figure.

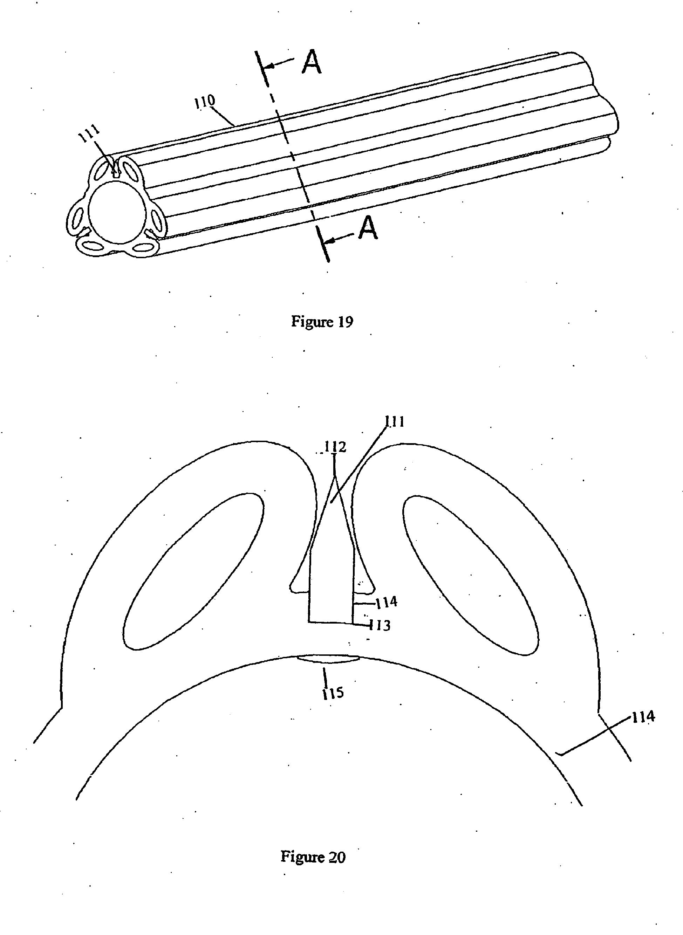

Figure 19 shows a perspective view of a further possible sheath/treatment implement construction.
Figure 20 shows a cross-sectional view of the construction of Figure 19 taken along the line A-A in Figure 19.

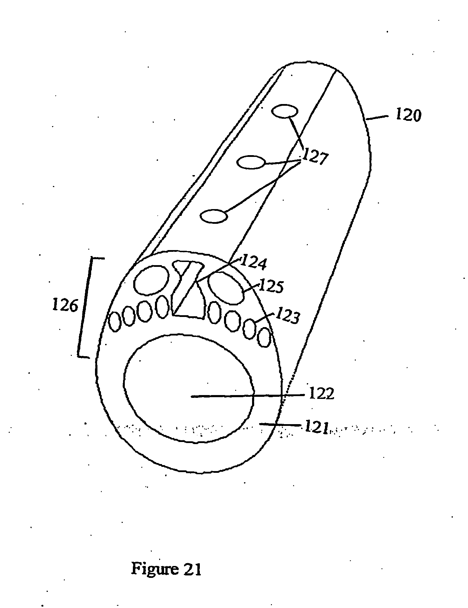

Figure 21 shows a perspective view of a further possible construction of a sheath of the present invention adapted to house internally (in a pocket) an implement such as a needle.

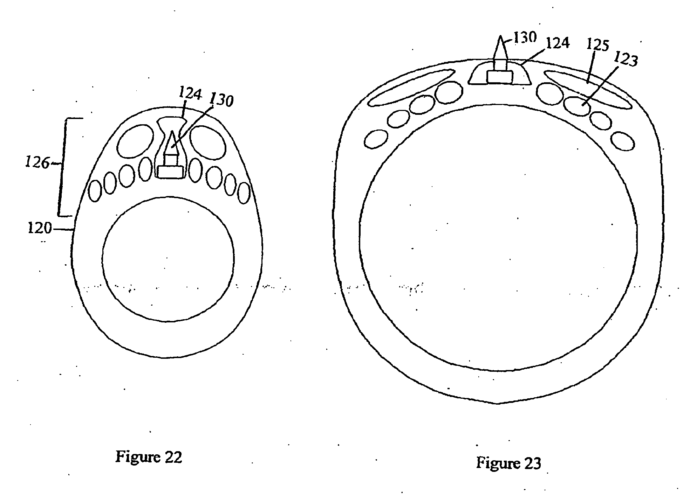

Figure 22 shows a cross-sectional view of a sheath according to Figure 21 in an unexpanded configuration and having implements mounted therein.
Figure 23 shows a cross-sectional view of a sheath according to Figure 21 in an expanded configuration with implements in a working position

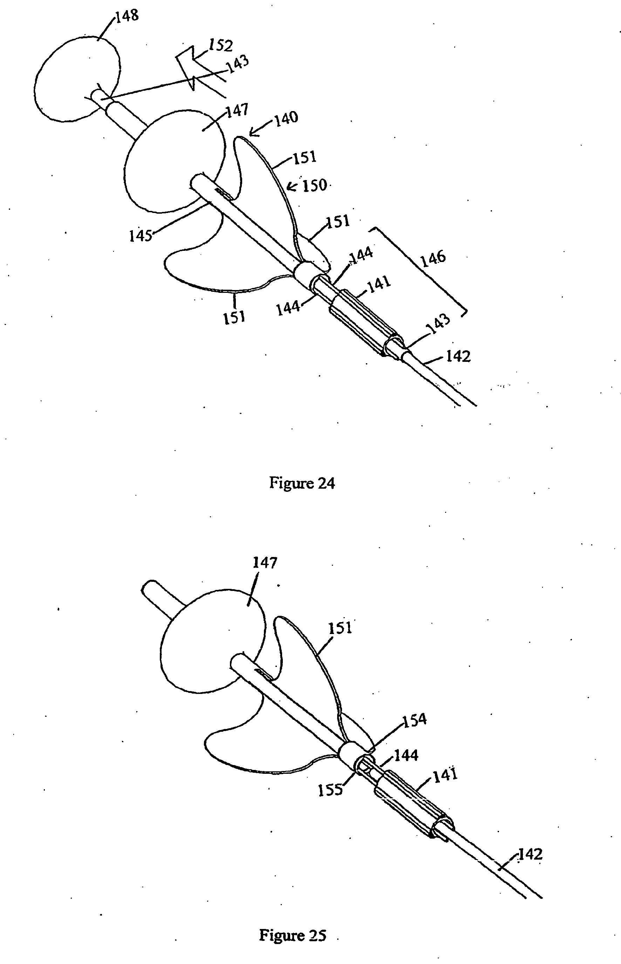

Figure 24 shows a perspective view of one example of a sheath loading device in position to place a sheath over a balloon catheter.
Figures 25 through 27 show the sequence for transferring the sheath from the loading device onto the catheter.

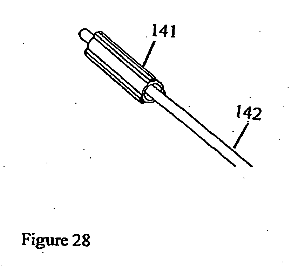

Figure 28 shows a perspective view of the loaded catheter.

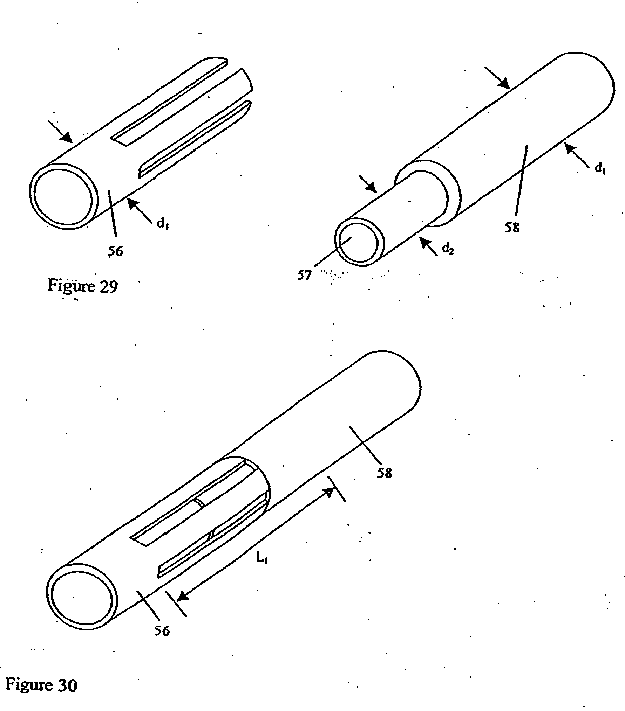

Figure 29 shows two parts of a sheath loading device.
Figure 30 shows the parts from Figure 29 assembled.

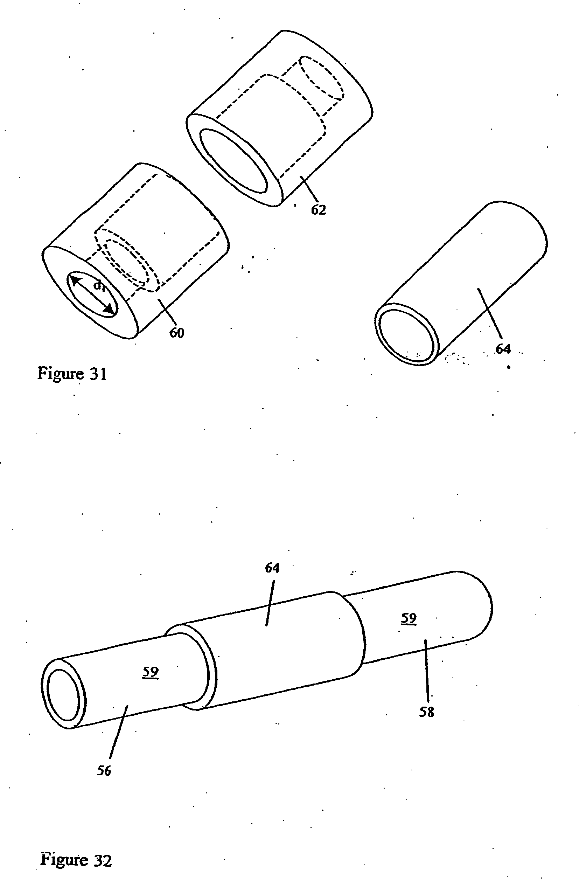

Figure 31 shows additional parts of a sheath loading device.
Figure 32 shows the parts of Figures 29 and 30 on which a sheath is mounted.

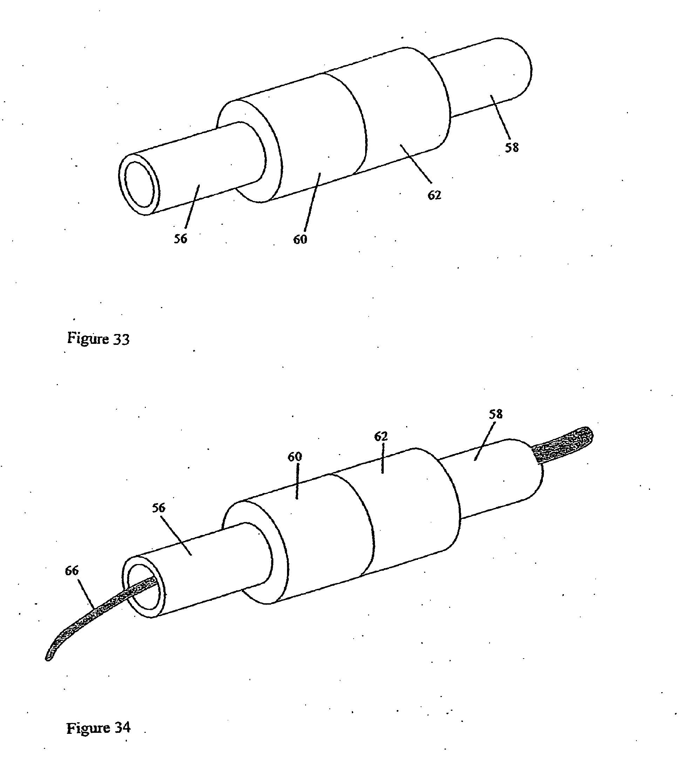

Figure 33 shows the fully assembled sheath loading device.
Figure 34 shows the sheath loading device *in situ* on a balloon catheter prior to loading.

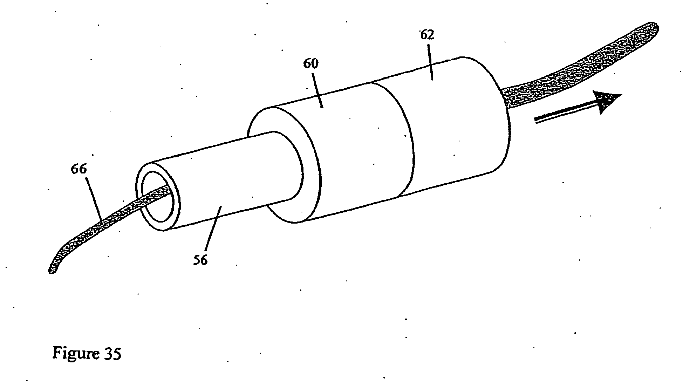

Figures 35 to 38 show the stages of disassembly of the sheath loading device as a sheath is loaded onto a catheter balloon.

### Detailed Description of the Drawings

Presented in the drawings is a catheter based device for the treatment of internal body cavities such as arteries/veins or other hollow organs. Also presented is a retrofit sheath and a sheath loading device.

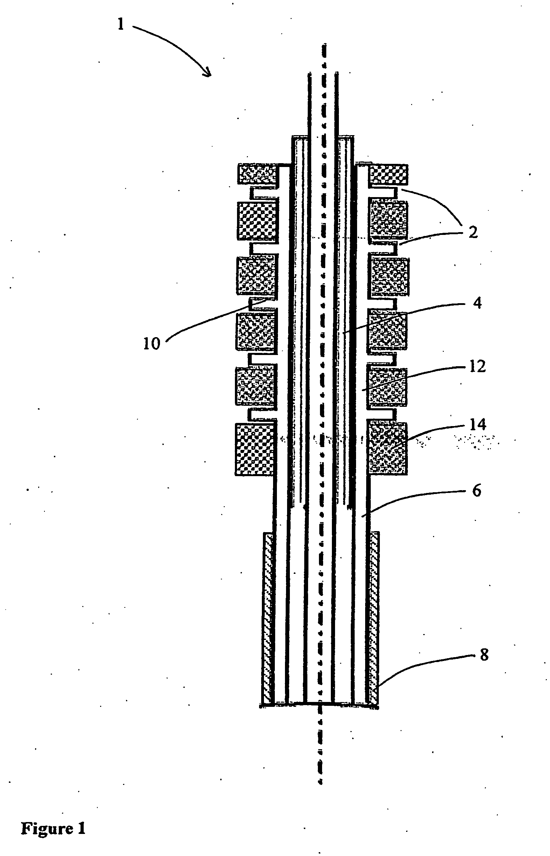

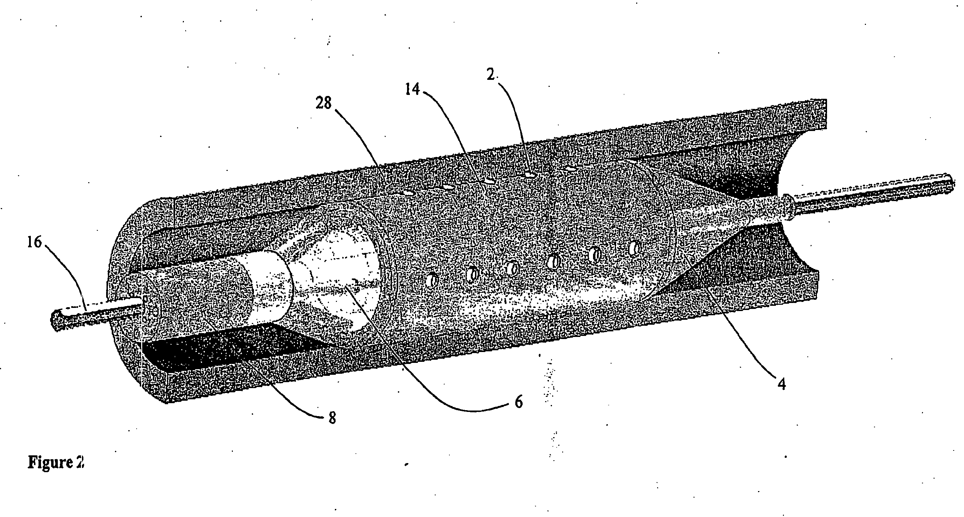

Figure 1 shows a catheter based drug delivery device 1. The device is insertable into a vasculature via a guide wire (as shown in Figure 2), includes micro-needles 2 that have two positions, a retracted position and an extended position. These needles 2 are mounted on the surface of a balloon catheter 4 and connected via flexible tubing 6 to a multi-lumen supply hose 8. The needles/micro-needles or stems (for directly injecting medicine(s)) attached to hollow needle base reservoirs 12. The needle stems 10 project outward from the reservoir 12 and are protected within a rubber sleeve or sheath 14. Upon inflation of the balloon 4 the needles 2 move outwards (in the radial direction), and stretching and compressing of the protective sheath 14 occurs, which in turn acts to expose the needles 2. The needles 2 when exposed can become embedded in the wall of the body cavity. Drugs may be delivered locally, for example to the diseased vessel wall, when the balloon 4 is inflated and subsequently when the needles 2 are embedded in the body cavity such as an artery wall. When balloon deflation occurs the needles 2 retract under the canopy of the sheath 14. The deflated assembly can now be safely removed from the body via a guide wire 16. During this procedure, the needles 2 are concealed and will not cause damage to the endothelium upon insertion and removal of the device.

At rest, the inner diameter of the elasticised sheath 14 is dimensioned so as to be smaller than the outer diameter of a balloon catheter 4 in its collapsed state. This ensures a tight fit between the sheath and balloon at all times when the sheath is loaded on the balloon. The sheath must therefore be stretch-fitted onto the balloon catheter. The elastic nature of the sheath ensures that the sheath will exert a compressive force on the balloon at all times. The balloon is thus maintained in its deflated state at all times except when a greater opposite force is exerted on the sheath by the balloon under the influence of air/fluid introduced under pressure into the balloon to inflate it.

In all examples the expandable member (balloon) will generally have a collapsed configuration where there is substantially no air or other inflating fluid in the balloon. Generally the balloon will also be in a folded configuration when collapsed. Desirably the compressive force of the sheath acts on the balloon in its folded configuration. The sheath acts to bias the balloon toward its folded configuration.

When the balloon is inflated, it is desirable that the sheath causes a tight seal between the needles and the artery wall allowing leak-free delivery. This seal may be achieved by selecting a soft material for the sheath such as a silicone material. Other suitable materials for the sheath include polyurethane and rubber.

As mentioned, elastic properties of the sheath cause the needles to retract once the balloon is deflated. This allows the device to return to its original configuration and allows the device to be used at multiple sites during the one procedure.

The protective foam-rubber cover or sheath 14 is shown in Figure 2. The selected material is both flexible and compressible enough to allow the needle stems 2 to expose upon balloon deployment, but more importantly provides and aids timely retraction and protection of the needle stems when balloon deflation occurs. This is particularly important for safe insertion and timely removal of the device.

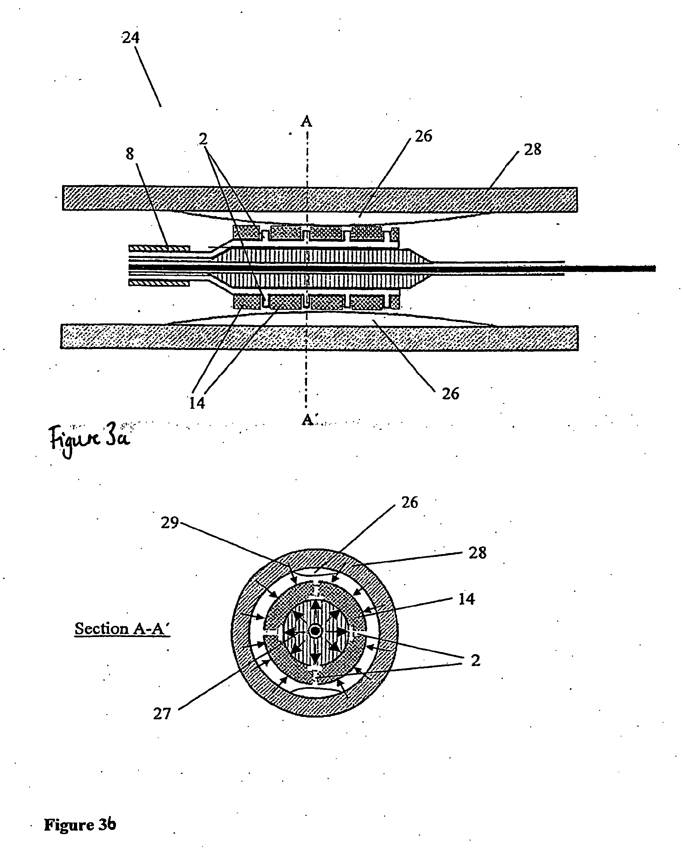

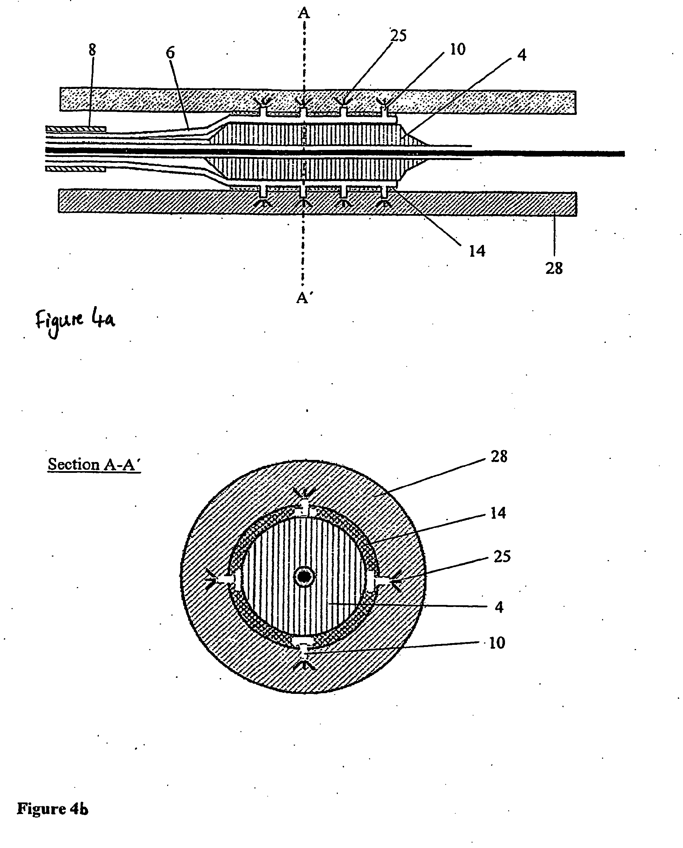

Figures 3 and 4 depict sectional schematics of the device during operation and in-vivo, within a partially occluded vascular cavity 24. In Figure 3, a plaque 26 is shown to have occurred locally around the inner cavity wall 28 causing partial occlusion. The device is shown placed in situ. Arrows 27 represent the balloon deployment force while arrows 29 represent the reaction force of the compressing sleeve.

Figures 3 and 4 illustrate one of the key features of the device which is shown in operation during mid and post deployment. As shown, the balloon pressure 27 causes the micro-needles 2 to move outward in the radial direction. Due to the compressive force and the circumferential stretch the protective sheath 14 is compressed (generally compression of the sheath will be due to the Poisson effect) thus exposing the micro-needle stems 10 allowing drug delivery (indicated by lines 25) directly into the plaque 26 on the cavity wall 28.

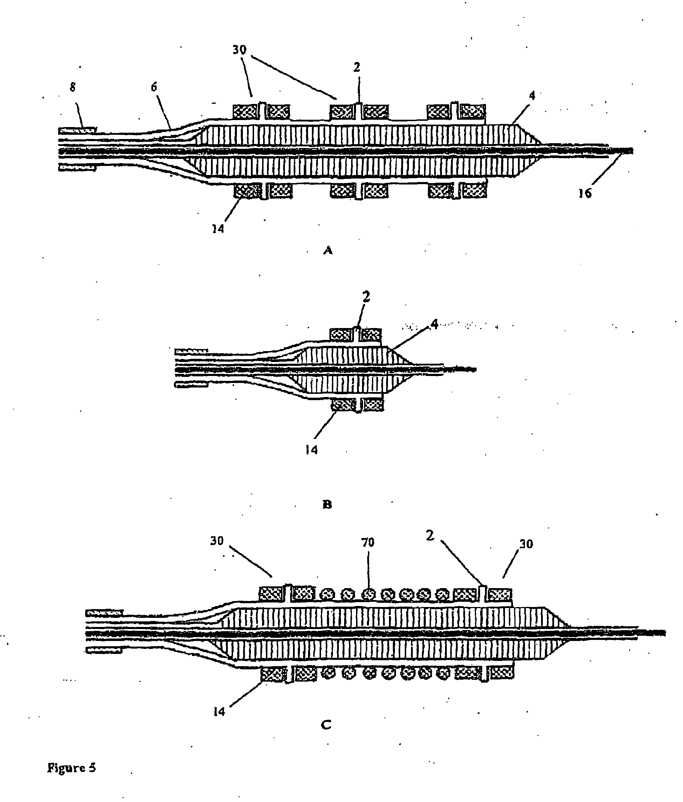

Figure 5 depicts three examples of devices, labeled A-C. Example A is a particularly flexible embodiment based on a modular design where the sheath 14 is provided with a plurality of rings 30 of material. These rings 30 may be completely separate from one another or may be connected by one or more interconnecting links. Example B has a short balloon 4, and the sheath 14 comprises treatment implements 2 adjacent the balloon's leading end. This example is most suitable to treating chronic total occlusions, as therapeutic delivery will occur as close as is possible to the blockage. This ensures that the therapeutic solution could instigate remodeling of the vasculature as close as possible to the diseased section, for example angiogenesis promoters would allow collaterals to form immediately upstream of the blockage ensuring that all areas of the limb/organ are supplied with blood flow. Embodiment C is a proximal and distal restenosis module suitable for attachment to a stent-loaded catheter. A stent 70 is shown in situ around the central portion of the balloon 4, between the sheath rings 30 which are confined to either end of the balloon 4. This module has the capability to deliver therapeutic agents to the artery wall immediately distal, proximal or both, of the area where a stent is being implanted, this would remove or reduce the risk of edge restenosis.

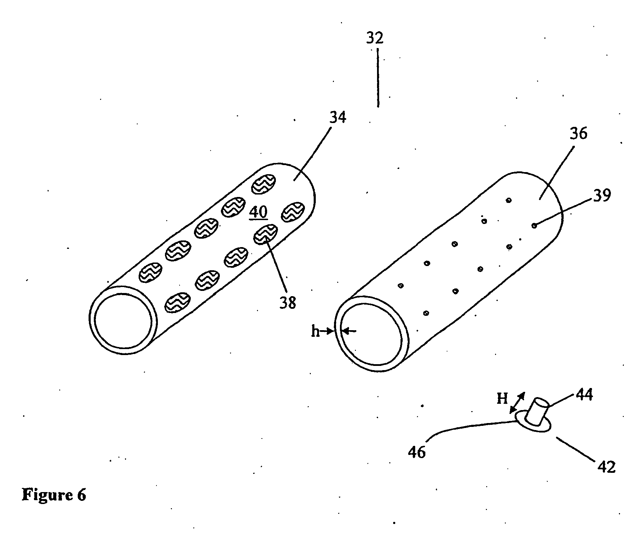

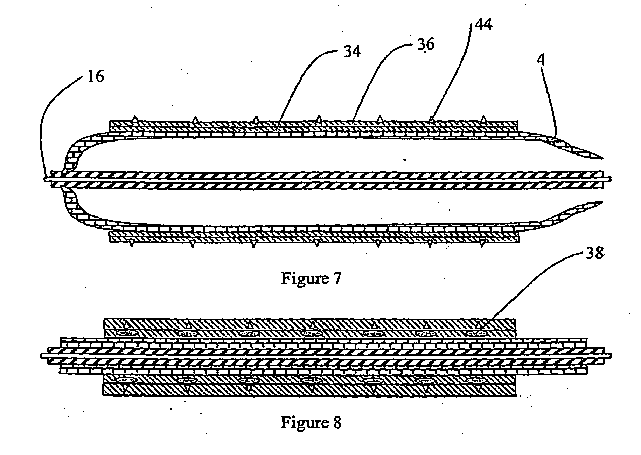

Figure 6 shows a retrofit sheath 32 according to one embodiment of the invention. The sheath is a two part sheath comprising an inner 34 and outer sheath 36. The inner sheath 34 has concave reservoirs 38 in (for example molded into) its outer surface 40, while the outer sheath 36 has small holes 39 defined within it to allow the needles sit within. A needle/plate assembly 42 sits beneath the outer sheath 36. The height h of the outer sheath 36 is greater than the height H of the needles 44. Once the needle assemblies 42 are in place, with the plates 46 positioned over the concave reservoirs, the outer sheath 36 is mounted over the inner sheath 34 to form the completed sheath shown in Figures 7 and 8. When the sheath is loaded on the catheter the therapeutic solution is stored within the sheath in the concave cavities/reservoirs. After the catheter has been maneuvered to the site of vascular disease the balloon is dilated. Upon dilation of the balloon, the sheath is stretched and the cavities within the sheath reduce in volume. This decrease in volume causes the therapeutic solution to be expelled from the reservoir and delivered to the site of disease.

Figure 7 shows the retrofit sheath of Figure 6 loaded onto a balloon catheter, the balloon catheter in its expanded configuration. Figure 8 shows the same arrangement with the catheter in its retracted configuration.

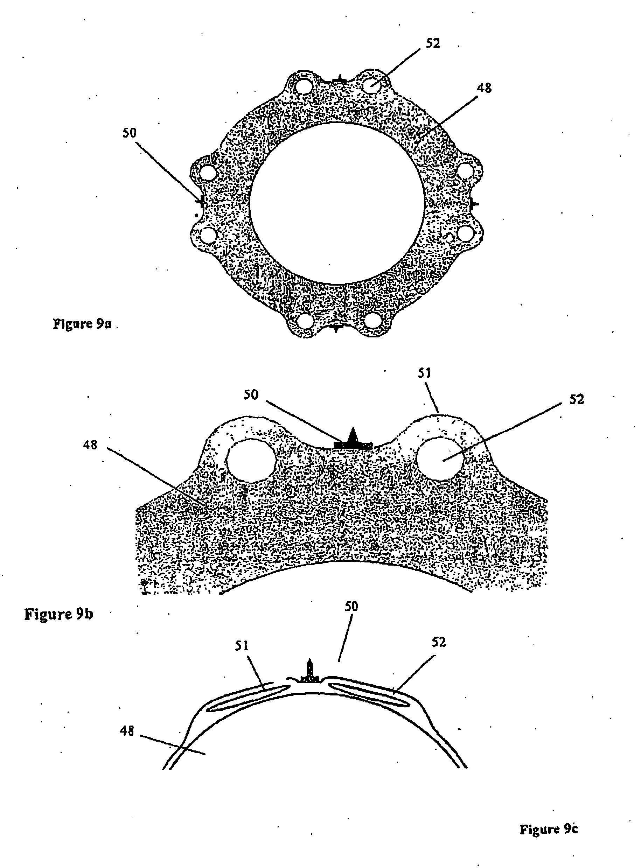

In the case of a retro fit sheath to be used as a cutting device, a simplified sheath may be employed. Figures 9a-9c show a retrofit cutting sheath 48 wherein the treatment implements are blades 50, which may be microsurgical scalpels. The scalpels are initially concealed from the artery wall by the external contours of the sheath 48, this allows the catheter to be navigated to the diseased portion of an artery without damaging the healthy vessel wall. It is the protuberances or bumps 51 in the sheath 48 as shown in Figure 9, which allow the blades 50 to be concealed from the artery wall, prior to and after use. When the sheath is positioned on a balloon and the balloon is inflated, the holes 52 in the bumps 51 flatten out as shown in Figure 9c, the contours of the sheath 48 become smooth and the cutting edges of the blades 50 are exposed. These blades 50 then contact the stenotic artery wall and allow an atherosclerotic lesion to be dilated in a controlled fashion. This approach allows the balloon expansion force to be concentrated at a number of discrete points and difficult lesions can be dilated successfully at lower pressures (4-8 atm or 4 - 8 x 10⁵ Pa).

The sheath or sleeve 50 can be adapted to be retrofitted to any balloon catheter. In the case of the present invention the sheath 48 is made of an elastic material and it will be appreciated that concealment of implements 50 is achieved because of the elastic properties of the sleeve 48. The holes 52 in the sheath will allow exposure of the blades 50 upon dilation of the balloon and deformation of the sheath, this is shown in the final schematic of Figure 9. There could be many other designs of cutting sheath, including a discontinuous tube that is joined at discrete points, similar to the needle version shown in Figure 5a.

Figure 10 shows a perspective (truncated) view of a sheath 80. The sheath 80 is suitable for fitting to a balloon catheter 90 of the type shown in Figure 11. The balloon catheter 90 has an expandable portion 91 which in the embodiment is an inflatable balloon. When the sheath 80 is over fitted to the catheter 90, it takes the form of the assembled configuration /device 100 shown in the Figure 12. Flexible microblades (in the embodiment 3 of them) of the type shown in Figure 13 have been attached to the sheath 80 between respective pairs of protuberances on the sheath 80 as will be described in more detail below. While the present example is described as having cutting blades it will be appreciated that the sheath could carry alternative of additional treatment implements. The blades 90 have a cutting tip 96 and a base end 97. The base end 97 is attached to the sheath 80 by adhesion though alternative methods of attachment can be utilised. In the example the blades 95 run substantially the entire length of the sheath to provide a cutting action along the length of the balloon. The blade is made of flexible material and is substantially continuous. It will be appreciated that the blade may be formed in a series of shorter blade segments.

The configuration of the device shown in Figure 12 shows the contracted configuration of the balloon. In this configuration the device is adapted to travel within a body lumen or vessel to a target area as the implements are shielded within the sheath.

A number of protuberances are formed as part of the sheath 80. Each protuberance is in the form of elliptical protuberance 81 each with a hollow internal pocket 82 In the embodiment the pockets 82 run along substantially the entire(working) length of the sheath 80 and formed on annular ring 85 of the sheath. It will be appreciated however that as the protuberances shield the implements from contact with the lumen when the device is being moved for travel within the lumen, the length and position of the protuberances can be adjusted according to be required shielding function. Each pocket 82 is hollow being formed by a fold of sheath material.

As can be seen from the drawings, the protuberances are provided in pairs. In the embodiment there are three pairs of protuberances. Each one of a given pair are on opposing sides of the treatment implements. In the embodiment each of the protuberances has a curved exterior surface 84. The surface 84 is convex in the shape. As can be seen from the drawings of the protuberances are substantially elliptical in cross-sectional shape. Each pair of protuberances converge towards each other (along their major axes) to any point above the working implements. In this way, the protuberances are profiled (so the highest point is) toward the working implements to ensure that each working implement is effectively shielded (laterally). In this configuration the working implements are nested within the protuberances.

As shown in Figure 14, a nose cone may be provided to smooth the transition between the catheter 90 and the sheath 80. It will be appreciated that the term "nose cone" is used to indicate any suitable nose portion that provides such a transition, and is not limited to conical shapes. Desirably the nose cone has a tapered profile. The nose cone is provided on the leading end 102 of the catheter/assembled catheter and sheath. In the embodiment, the nose cone is a flared skirt 103 which provides a smooth surface transition between the catheter tip 102 and the sheath 100. The nose cone may be optionally adapted to match the exterior profile of the sheath including its protuberances. In the embodiment this is shown by having raised portions 104 which are joined by (lower) transitional portions 105. It will be appreciated that a similar device may be provided on the opposite end of the assembly 100 and in an analogous fashion. In such a case the second device may be considered a "tail cone". It will assist in retraction of the device from the body (being on the trailing end of the assembly).

Figures 15-17 show the change in configuration of the sheath during expansion of the balloon of the catheter. The catheter has been omitted from the drawing for the purposes of clarity. However the expansive force being exerted (internally) on the sheath comes from the balloon catheter.

Initially, as shown in Figure 15, in the unexpanded configuration the working implement 95 is shielded within pairs of protuberances. As shown in the drawings, there are three working implements, each spaced approximately 120° degrees apart about the sheath 80. In this configuration, the assembled sheath/catheter can travel within a body lumen without fear of the implements 95 snagging.

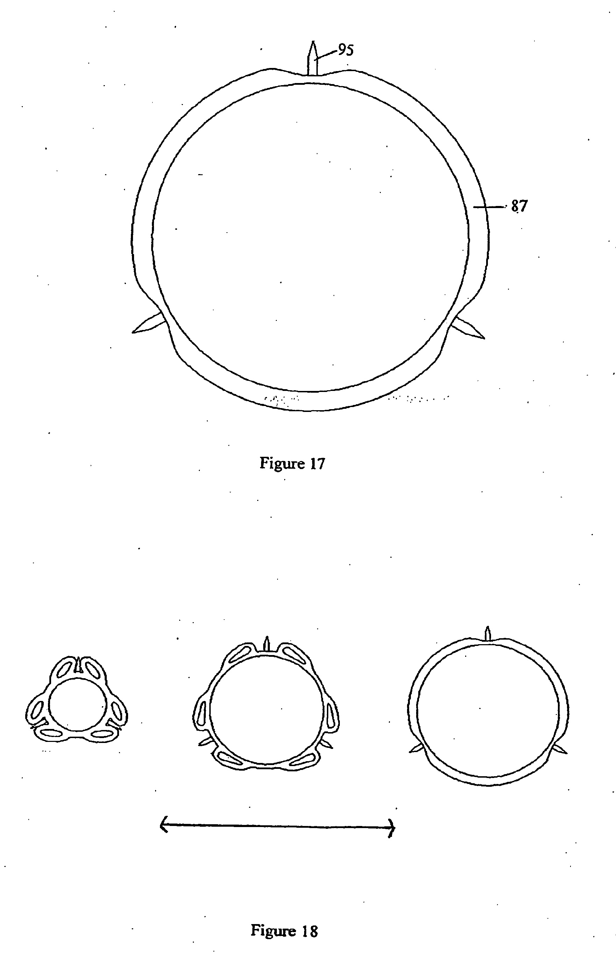

As Figure 16 demonstrates, as the expansive pressure exerted from within by the balloon of the catheter is taken up by the sheath 80, the thickness of the sheath decreases to expose the implements for contact with the target area of a vessel wall. As can be seen from Figure 16 the protuberances 81, and in particular the pockets 82, begin to flatten out so that the effective thickness of the sheath 80 is substantially reduced. The effect is then that the implement 95 and (in particular the cutting tip 96) is urged out of its nested position between opposing protuberances and is no longer shielded from contact with a vessel wall. The annular ring 85 reduces in thickness, and the protuberances 81 both reduce in thickness and begin to flatten (both effects contributing to exposure of the implement). Indeed as expansion continues, as Figure 17 shows, the protuberances may flatten and stretch to the extent that they are essentially assimilated into one larger (circular) stretched ring 87. In the configuration of Figure 16 or Figure 17 (or intermediate positions) the implements are available to be worked. Contraction occurs when the balloon is deflated and in reverse to the position in relation to expansion described above.

Figure 18 is provided for convenience showing the reversible sequence of sheath configurations during expansion (left to right) and contraction (right to left).

It is clear that once the device returns to its contracted configuration, it is again free to move within the body without fear of snagging etc.

Figure 19 shows a sheath 110 which is similar in construction to sheath 80 described above. In this case the treatment implements, (blades 111), are shown in the shielded position with the tip 112 of the blade shielded from contact with the body. One of the additional features as compared to the sheaths described above is that the working implement (blade 111) is recessed into the sheath. In particular, the base portion 113 extends through the surface of the sheath and is embedded in the sheath. The implement can be In the embodiment shown, the base portion thereof accommodated within the recess. In the embodiment shown, the implement is moulded into position when the sheath is being formed. Alternatively, the channel or other such recess could be provided in the sheath to which the implement is later attached. To avoid possible dislodgement of the implement from its recessed position, it may be prudent to provide a stiffening member proximate the implement to inhibit the ability of the sheath to stretch at or about the point of fixing of the implement to the sheath. In the embodiment, a stiffening member 115 extends along the sheath at a position beneath the implement 112. The stiffening member 115 is thus of sufficient length to inhibit dislodgement of the implement at any given point.

Figure 21 shows another alternative embodiment of the present invention. The sheath 120 is shown in its unexpanded configuration. The sheath 120 has an annular ring of material 121. The aperture 122 is for receiving a catheter balloon such as described above. A series of pockets are formed in the sheath 120. In particular, the sheath 120 has a deformable head portion 126 which is provided with a number of pockets. In the embodiment only one head portion is shown, but it will be appreciated that a plurality could be provided, for example such head portion constructions could be replicated in other parts of the sheath. The pocket 124 is for housing an implement within it A series of pockets 123 are provided on either side of the implement pocket 124. Further, larger pockets 125 are also provided on opposing sides of the implement pocket 124. In the embodiment, the sheath 120 is formed with apertures 127 these apertures are arranged to be located over the working implement, which in the embodiment is desirably a needle. Exposure of the implement occurs through the apertures 127 as will be described in detail below.

Figure 22 shows an end view of the sheath 120 having an implement, in the embodiment a needle 130, housed within the pocket within the sheath. In particular, the needle 130 is within the implement pocket 124. It will be appreciated that a plurality of implements, such as a plurality of needles 130, could be provided, for example for exposure through apertures 127. For the sake of clarity however, the action of one needle 130 is being described here. The configuration in Figure 22 is the unexpanded configuration with the implement shielded by the sheath.

Figure 23 shows the expanded configuration with the sheath 120 having been expanded under the force of an expanding balloon. As can be seen clearly from the figure, under the expansive force, the thickness of the sheath has decreased. This has occurred due to stretching of the sheath itself and also due to flattening of (all of) the pockets of the sheath. In particular, it will be appreciated that the head portion 126 of the sheath has now substantially reduced in thickness. The effect, has seen from Figure 23, is that the needle 130 has been pushed out through an aperture 127 so that it is now in a working configuration. Fluid can be delivered to the needles as described above for other embodiments.

Figures 24-28 show an example of a loading device 140. The loading device is for loading a stretchable tubular sheath 141 onto a balloon catheter. The loading device has a stretching portion 146. The stretching portion 146 comprises a plurality of members, which in the embodiment are fingers 144. The fingers are expandable relative to each other to stretch of the sheath. Figure 24 shows the stretched configurations of the sheath, with the fingers 144 having being inserted within the sheath and having been moved apart by the insertion of the push rod 143. The push rod 143 is provided with a handle 148 for ease of manoeuvre. The push rod 143 is of a hollow tubular configuration. It can therefore slidingly accommodate a catheter 143 therein. It is desirable that the push rod 143 is transparent or is otherwise provided with an indicator to allow correct positioning of the balloon relative to the sheath. In this respect "transparent" means sufficiently translucent to allow the position of the catheter to be visually determined, or including one or more open windows through which the catheter can be viewed.

The fingers 144 are mounted in a mounting portion 145. The fingers are retractable as will be described below, by their associated grips 151. In the embodiment three fingers 144 are provided, each approximately 120° apart from the next. As indicated in Figure 24, by pulling the push rod 143 in the direction of arrow 152, the rod 143 is retracted from between the fingers 144. The result of removal of the push rod 143 is the configuration shown in Figure 25.

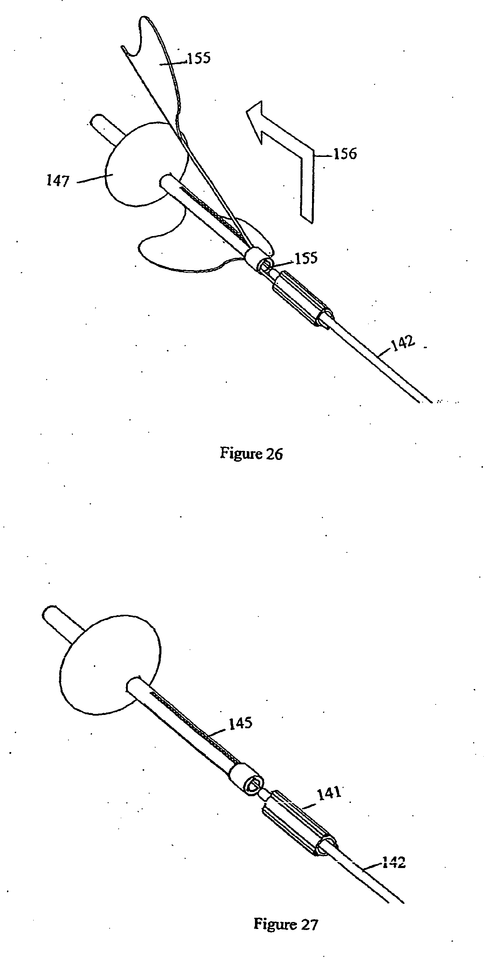

The next stage in the process which is shown in Figure 26, is the removal of the fingers 144. This is done by gripping a handle 147 of the mounting portion 145 which mounts three fingers. The three fingers are maintained in a spaced apart configuration by a guide 154. The guide 154 has apertures 155 therein through which the fingers extend. As shown in Figure 26 the fingers 144 can be retracted by pulling on the grips 155 as indicated by arrow 156. This pulls the fingers 144 out from under the sheath and releases the sheath onto the catheter 142. This leads to the configuration of Figure 27 where the fingers are no longer underneath the sheath. That means that the mounting portion can be taken away to leave the sheath 141 in place on the catheter 142. The catheter 142 with the sheath 141 loaded thereon is shown in Figure 28. It will be appreciated that the expanding members (the fingers) are flexible.

Figures 29 to 34 show the assembly process of a further sheath-loading device 54. The assembled device consists of four interconnecting parts.

As shown in Figure 29, the first part is a tube 56 with slots machined on part of its length. The second part is a tube 58 with two different outer diameters, d₁, and d₂. The diameter d₁ is the same as the diameter d₁ of tube 56. The internal diameter of tube 58, defined by inner surface 57 will be large enough to fit balloon catheters through.

Figure 30 shows the first and second parts assembled with tube 58 being pushed into the end of tube 56. The length L₁ is the position that the sheath with implements will be placed.

In Figure 31, the third and fourth parts are shown as two tubes 60, 62. Tubes 60, 62 fit over the assembled tubes 56 and 58 when the sheath is mounted on them. Tubes 60, 62 when assembled have functions: (i) to protect the users hands from the implements when the sheath is being placed on a balloon catheter, (ii) to hold the sheath in place when tubes 60 and 62 are being removed during the sheath mounting procedure. Tubes 60 and 62 can screw together to form one part or be press fit together. The smallest internal diameter of parts 60 and 62 is equivalent to the diameter d₁ of tubes 56 and 58. Also shown in figure 12 is a sheath 64 with implements (not shown) on it. Its unstrained diameter will be at least 10% less than the diameter of the balloon catheter that it will be mounted on.

Figure 32 shows assembled parts 56 and 58 with the sheath 64 mounted their outer surface 59. The sheath 64 is stretched in the circumferential direction to fit on these assembled parts.

Figure 33 shows the complete assembly with parts 60 and 62 loaded over the sheath 64. This is how the product could be delivered to the customer.

Figures 34 to 38 show the sheath-loading process. As shown in Figure 34, a balloon catheter 66 is placed within the inner tubes 56 and 58 of the complete assembly

As shown in Figure 386, to begin disassembly of the assembly, parts 60 and 62 are held by the operator and part 58 is pulled back in the direction of the large arrow and removed from the catheter. This allows the slotted end of part 56 to drop onto the balloon catheter 66 and relieve some of the pressure that the sheath exerts on part 56. It also make it easier to remove part 56.

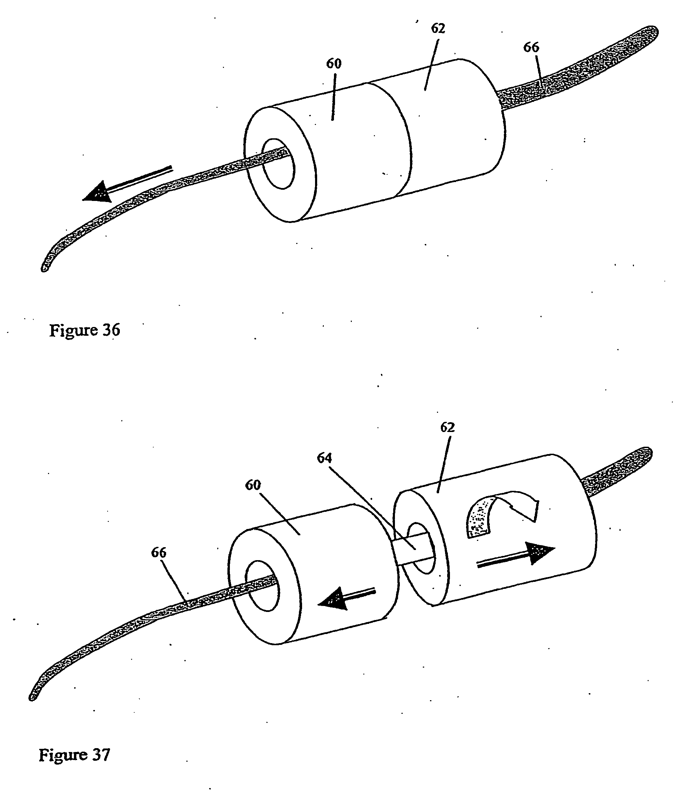

As shown in Figure 36, parts 60 and 62 are then held by the operator and part 56 is pulled in the direction of the large arrow, and removed from the catheter.

The final step is shown in Figure 37. Parts 60 and 62 are twisted and pulled apart in the direction of the large arrows, and removed from the catheter.

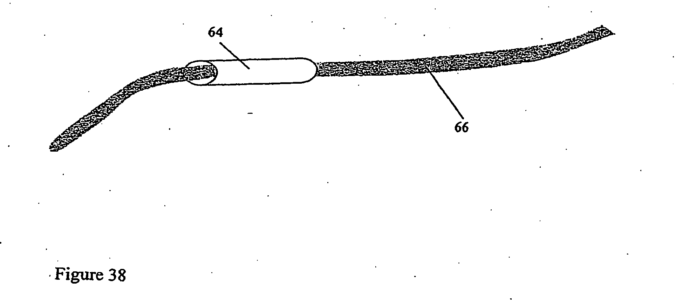

Figure 38 is a simple schematic showing the sheath 64 loaded on the balloon catheter 66, the sheath 64 compressing the balloon.

Parts 56, 58, 60 and 62 can be made of any material, however the most preferable material would be a transparent material, so that the operator can see where the sheath is being mounted.

Radiopaque markers could be added to the sheath to aid placement during the procedure (balloon angioplasty procedure).

An extra part may be needed to hold the catheter in place before part 58 is removed or this could be incorporated into part 58. Otherwise an extra hand is needed.

Part 56 can be tapered as well to make removal easier. Parts 56 and 58 may be lubricated to make their removal easier. All parts of the loading device may be provided with ergonomically designed grips to aid control.

It is appreciated that certain features of the invention, which are, for clarity, described in the context of separate embodiments, may also be provided in combination in a single embodiment. Conversely, various features of the invention which are, for brevity, described in the context of a single embodiment, may also be provided separately or in any suitable sub-combination.

## Claims

1. A sheath (80, 110) for fitting to a balloon catheter for treating a target area of a vessel wall of a vessel within a human or animal body, the sheath having a contracted and an expanded configuration and comprising:
at least two spaced apart treatment implements (111) mounted within the sheath so as to extend radially outwardly from the sheath, wherein in the contracted configuration the implements are shielded within the sheath, and in the expanded configuration the thickness of the sheath decreases to expose the implements for contact with the target area of the vessel wall, **characterised in that** a base end (113) of each treatment implement (111) is recessed into the sheath.

2. The sheath of claim 1 further comprising at least one stiffening member (115) proximate each treatment implement to inhibit the ability of the sheath to stretch at or about the point of fixing of the treatment implement to the sheath.

3. The sheath of claim 2 wherein said at least one stiffening member (115) extends along the sheath at a position beneath the treatment implement.

4. The sheath of any preceding claim wherein the treatment implements (111) are cutting implements.

5. The sheath of any preceding claim further comprising at least one protuberance (81) on the sheath's outer surface; wherein each protuberance has a hollow internal pocket (82), wherein in the sheath's expanded configuration a deformation of the sheath causes the pocket to flatten out thereby reducing the size of the protuberance in the radial direction to expose each treatment implement.

6. The sheath of any preceding claim further comprising at least one pair of protuberances (81), each on opposing sides of each treatment implement; and wherein the at least one pair of protuberances converge toward each other and to a point above the treatment implement.

7. The sheath of any preceding claim, wherein the sheath takes the form of an annular ring of material (121) and within the ring at least one hollow internal pocket (123, 124, 125) is formed, wherein, in the sheath's expanded configuration, the deformation of the sheath causes the pocket to flatten out.

8. A sheath according to Claim 7 wherein a treatment implement is housed within at least one hollow pocket (124), and in the balloon's expanded configuration, the deformation of the sheath causes the pocket to flatten out so as to expose the treatment implement for use.

9. A sheath according to Claim 8 wherein the pocket (124) is provided with an aperture (127) through which the treatment implement extends in the balloon's expanded configuration.

10. A sheath according to Claim 8 or 9 wherein a plurality ofpockets (124) are provided, each housing a treatment implement.

11. A sheath according to any one of Claim 8 to 10 wherein at least one pocket (123, 125) is provided which does not house a treatment implement.

12. A sheath according to Claim 11 wherein a plurality of pockets (123, 125) are provided each of which does not house a treatment implement.

13. The sheath of any preceding claim further comprising a nose cone arranged to provide a transitional profile between a leading end of the sheath and a balloon catheter when the sheath is loaded thereon.

14. The sheath of claim 13 wherein the nose cone is adapted to match the exterior profile of the sheath.

15. The sheath of any preceding claim further comprising a tail cone arranged to provide a transitional profile between a trailing end of the sheath and a balloon catheter when the sheath is loaded thereon.

## Patentansprüche

1. Mantel (80, 110) zur Befestigung an einem Ballonkatheter zur Behandlung eines Zielgebiets einer Gefäßwand eines Gefäßes innerhalb eines menschlichen oder tierischen Körpers, wobei der Mantel eine verengte und eine aufgeweitete Konfiguration aufweist, und vorgesehen sind:
zumindest zwei beabstandete Behandlungsgeräte (111), die so innerhalb des Mantels angebracht sind, dass sie sich in Radialrichtung nach außen von dem Mantel aus erstrecken, wobei in der verengten Konfiguration die Geräte innerhalb des Mantels abgeschirmt sind, und in der aufgeweiteten Konfiguration die Dicke des Mantels abnimmt, um die Geräte zum Kontakt mit dem Zielgebiet der Gefäßwand freizulegen, **dadurch gekennzeichnet, dass** ein Basisende (113) jedes Behandlungsgeräts (111) in den Mantel eingebaut ist.

2. Mantel nach Anspruch 1, welcher weiterhin zumindest ein Versteifungsteil (115) in der Nähe jedes Behandlungsgeräts aufweist, um die Fähigkeit des Mantels zu sperren, sich am Punkt der Befestigung des Behandlungsgeräts an dem Mantel oder um den Punkt herum zu strecken.

3. Mantel nach Anspruch 2, bei welchem das zumindest eine Versteifungsteil (115) sich entlang dem Mantel an einem Ort unterhalb des Behandlungsgeräts erstreckt.

4. Mantel nach einem der voranstehenden Ansprüche, bei welchem die Behandlungsgeräte (111) Schneidgeräte sind.

5. Mantel nach einem der voranstehenden Ansprüche, welcher weiterhin zumindest einen Vorsprung (81) auf der äußeren Oberfläche des Mantels aufweist; wobei jeder Vorsprung eine hohle innere Tasche (82) aufweist, wobei in der aufgeweiteten Konfiguration des Mantels eine Verformung des Mantels dazu führt, dass die Tasche abgeflacht wird, wodurch die Größe des Vorsprungs in Radialrichtung verringert wird, um jedes Behandlungsgerät freizulegen.

6. Mantel nach einem der voranstehenden Ansprüche, welcher weiterhin zumindest ein Vorsprungspaar (81) aufweist, jeweils ein Vorsprung auf entgegengesetzten Seiten jedes Behandlungsgeräts; wobei die Mitglieder des zumindest einen Behandlungspaares zueinander zusammenlaufen und zu einem Punkt oberhalb des Behandlungsgeräts.

7. Mantel nach einem der voranstehenden Ansprüche, wobei der Mantel die Form eines Kreisrings aus Material (121) annimmt, und innerhalb des Ringes zumindest eine hohle innere Tasche (123, 124, 125) ausgebildet ist, wobei in der aufgeweiteten Konfiguration des Mantels die Verformung des Mantels dazu führt, dass die Tasche abgeflacht wird.

8. Mantel nach Anspruch 7, bei welchem ein Behandlungsgerät innerhalb zumindest einer hohlen Tasche (124) aufgenommen ist, und in der aufgeweiteten Konfiguration des Ballons die Verformung des Mantels dazu führt, dass die Tasche abgeflacht wird, um das Behandlungsgerät zum Einsatz freizulegen.

9. Mantel nach Anspruch 8, bei welchem die Tasche (124) mit einer Öffnung (127) versehen ist, durch welche sich das Behandlungsgerät in der aufgeweiteten Konfiguration des Ballons erstreckt.

10. Mantel nach Anspruch 8 oder 9, bei welchem mehrere Taschen (124) vorgesehen sind, die jeweils ein Behandlungsgerät aufnehmen.

11. Mantel nach einem der Ansprüche 8 bis 10, bei welchem zumindest eine Tasche (123, 125) vorgesehen ist, die nicht ein Behandlungsgerät aufnimmt.

12. Mantel nach Anspruch 11, bei welchem mehrere Taschen (123, 125) vorgesehen sind, die jeweils kein Behandlungsgerät aufnehmen.

13. Mantel nach einem der voranstehenden Ansprüche, welcher weiterhin einen vorderen Konus aufweist, der so ausgebildet ist, dass er ein Übergangsprofil zwischen einem Vorderende des Mantels und einem Ballonkatheter zur Verfügung stellt, wenn der Mantel dort angebracht wird.

14. Mantel nach Anspruch 13, bei welchem der vordere Konus angepasst an das Außenprofil des Mantels ausgebildet ist.

15. Mantel nach einem der voranstehenden Ansprüche, welcher weiterhin einen hinteren Konus aufweist, der so ausgebildet ist, dass er ein Übergangsprofil zwischen einem hinteren Ende des Mantels und einem Ballonkatheter zur Verfügung stellt, wenn der Mantel dort angebracht wird.

## Revendications

1. Gaine (80, 110) à assembler à un cathéter à ballonnet pour traiter une zone cible d'une paroi vasculaire d'un vaisseau au sein d'un organisme humain ou animal, la gaine présentant une configuration de type contraction et une configuration de type expansion et comprenant :
au moins deux instruments de traitement (111) séparés l'un de l'autre, installés à l'intérieur de la gaine de manière à s'étendre radialement vers l'extérieur depuis la gaine, où dans la configuration de type contraction les instruments sont protégés à l'intérieur de la gaine, et dans la configuration de type expansion, l'épaisseur de la gaine diminue pour exposer les instruments devant entrer en contact avec la zone cible de la paroi vasculaire, **caractérisée en ce qu'**une extrémité de base (113) de chaque instrument de traitement (111) est encastrée dans la gaine.

2. Gaine selon la revendication 1, comprenant en outre au moins un élément de renforcement (115) à proximité de chaque instrument de traitement afin d'empêcher que la gaine puisse s'étirer au niveau de ou aux environs du point de fixation de l'instrument de traitement à la gaine.

3. Gaine selon la revendication 2, dans laquelle ledit élément de renforcement (115), au moins au nombre de un, s'étend le long de la gaine à un emplacement qui se trouve en dessous de l'instrument de traitement.

4. Gaine selon l'une quelconque des revendications précédentes, dans laquelle les instruments de traitement (111) sont des instruments coupants.

5. Gaine selon l'une quelconque des revendications précédentes, comprenant en outre au moins une protubérance (81) sur la surface externe de la gaine ; dans laquelle chaque protubérance comporte une poche creuse interne (82), dans laquelle, dans la configuration de gaine de type expansion, une déformation de la gaine provoque un aplanissement de la poche et réduit ainsi la taille de la protubérance dans le sens radial pour exposer chaque instrument de traitement.

6. Gaine selon l'une quelconque des revendications précédentes, comprenant en outre au moins une paire de protubérances (81), chacune sur des faces opposées de chaque instrument de traitement ; et dans laquelle les protubérances de la paire de protubérances, au moins au nombre de une, convergent l'une vers l'autre et ce jusqu'à un point situé au-dessus de l'instrument de traitement.

7. Gaine selon l'une quelconque des revendications précédentes, dans laquelle la gaine prend la forme d'un anneau de matière de type annulaire (121) au sein duquel au moins une poche creuse interne (123, 124, 125) est formée, dans laquelle, dans la configuration de gaine de type expansion, la déformation de la gaine provoque un aplanissement de la poche.

8. Gaine selon la revendication 7, dans laquelle un instrument de traitement est logé à l'intérieur d'au moins une poche creuse (124), et dans la configuration de ballonnet de type expansion, la déformation de la gaine provoque l'aplanissement de la poche de manière à exposer l'instrument de traitement pour en permettre l'utilisation.

9. Gaine selon la revendication 8, dans laquelle la poche (124) est pourvue d'une ouverture (127) à travers laquelle s'étend l'instrument de traitement dans la configuration de ballonnet de type expansion.

10. Gaine selon la revendication 8 ou 9, dans laquelle une pluralité de poches (124) sont prévues, chacune logeant un instrument de traitement.

11. Gaine selon l'une quelconque des revendications 8 à 10, dans laquelle il est prévu au moins une poche (123, 125) ne logeant aucun instrument de traitement.

12. Gaine selon la revendication 11, dans laquelle il est prévu une pluralité de poches (123, 125), chacune d'entre elles ne logeant aucun instrument de traitement.

13. Gaine selon l'une quelconque des revendications précédentes, comprenant en outre une coiffe avant disposée de manière à fournir un profil de transition entre une extrémité d'attaque de la gaine et un cathéter à ballonnet lorsque la gaine est chargée dessus.

14. Gaine selon la revendication 13, dans laquelle la coiffe est adaptée pour correspondre au profil extérieur de la gaine.

15. Gaine selon l'une quelconque des revendications précédentes, comprenant en outre une coiffe arrière disposée de manière à fournir un profil de transition entre une extrémité arrière de la gaine et un cathéter à ballonnet lorsque la gaine est chargée dessus.
